# EUROPEAN PATENT SPECIFICATION

(11) **EP 2 206 788 B1**
(45) Date of publication and mention of the grant of the patent: **31.12.2014**
(21) Application number: 10158504.0
(22) Date of filing: 05.11.2004
(51) Int. Cl.: C12N 15/75, C12R 1/125, C12P 21/02

(54) **Recombinant microorganism**
Rekombinanter Mikroorganismus
Microorganisme recombinant

(30) Priority: 07.11.2003 JP 2003379167
(43) Date of publication of application: 14.07.2010
(62) Divisional of application: 04799676.4
(73) Proprietor: Kao Corporation, Chuo-Ku Tokyo 103-8210 (JP)
(72) Inventor: Tohata, Masatoshi, Tochigi 321-3426 (JP); Sawada, Kazuhisa, Tochigi 321-3426 (JP); Ozaki, Katsuya, Tochigi 321-3426 (JP); Kobayashi, Kazuo, Nara 630-0101 (JP); Ogasawara, Naotake, Nara 630-0101 (JP)
(74) Representative: Vossius & Partner

(56) References cited:
- DE-A1- 19 951 765
- WARNER JESSICA B ET AL: "CcpA-independent regulation of expression of the Mg2+-citrate transporter gene citM by arginine metabolism in Bacillus subtilis." JOURNAL OF BACTERIOLOGY, vol. 185, no. 3, February 2003 (2003-02), pages 854-859, XP002346326 ISSN: 0021-9193
- ALI NAIMA OULD ET AL: "Regulation of the acetoin catabolic pathway is controlled by sigma L in Bacillus subtilis" JOURNAL OF BACTERIOLOGY, vol. 183, no. 8, April 2001 (2001-04), pages 2497-2504, XP002346327 ISSN: 0021-9193
- WACKER INGRID ET AL: "The regulatory link between carbon and nitrogen metabolism in Bacillus subtilis: Regulation of the gltAB operon by the catabolite control protein CcpA." MICROBIOLOGY (READING), vol. 149, no. 10, October 2003 (2003-10), pages 3001-3009, XP002346328 ISSN: 1350-0872
- LANGBEIN INES ET AL: "Specific interaction of the RNA-binding domain of the Bacillus subtilis transcriptional antiterminator GlcT with its RNA target, RAT" JOURNAL OF MOLECULAR BIOLOGY, vol. 293, no. 4, 5 November 1999 (1999-11-05), pages 795-805, XP004461874 ISSN: 0022-2836
- EGETER O ET AL: "CATABOLITE REPRESSION MEDIATED BY THE CATABOLITE CONTROL PROTEIN CCPA IN STAPHYLOCOCCUS XYLOSUS" MOLECULAR MICROBIOLOGY, BLACKWELL SCIENTIFIC, OXFORD, GB, vol. 21, no. 4, 1996, pages 739-749, XP002916663 ISSN: 0950-382X
- LEBOEUF CELINE ET AL: "Characterization of the ccpA gene of Enterococcus faecalis: Identification of starvation-inducible proteins regulated by CcpA" JOURNAL OF BACTERIOLOGY, vol. 182, no. 20, October 2000 (2000-10), pages 5799-5806, XP002346330 ISSN: 0021-9193
- DATABASE Geneseq [Online] 29 January 2001 (2001-01-29), "Bacillus sp. KSM-S237 heat resistant alkaline cellulase DNA." XP002346334 retrieved from EBI accession no. GSN:AAA97493 Database accession no. AAA97493
- DATABASE EMBL [Online] 21 May 1993 (1993-05-21), "Bacillus sp. KSM-64 endo-1,4-beta-glucanase gene, complete cds; and unknown gene." XP002346335 retrieved from EBI accession no. EM_PRO:BSENDOGLU Database accession no. M84963

### SEQUENCE LISTING

<110> KAO CORPORATION
<120> Recombinant microorganisms
<130> M1728 EP/1 BLN
<150> JP 2003-379167
   <151> 2003.11.7
<160> 122
<170> PatentIn Ver. 2.1
<210> 1
   <211> 3150
   <212> DNA
   <213> Bacillus sp. KSM-S237
<220>
   <221> CDS
   <222> (573)..(3044)
   <223>
<220>
   <221> sig_peptide
   <222> (573)..(659)
   <223>
<220>
   <221> mat_peptide
   <222> (660)..()
   <223>
<400> 1
   gatttgccga tgcaacaggc ttatatttag aggaaatttc tttttaaatt gaatacggaa     60
   taaaatcagg taaacaggtc ctgattttat ttttttgagt tttttagaga actgaagatt    120
   gaaataaaag tagaagacaa aggacataag aaaattgcat tagttttaat tatagaaaac    180
   gcctttttat aattatttat acctagaacg aaaatactgt ttcgaaagcg gtttactata    240
   aaaccttata ttccggctct tttttaaaac agggggtaaa aattcactct agtattctaa    300
   tttcaacatg ctataataaa tttgtaagac gcaatatgca tctctttttt tacgatatat    360
   gtaagcggtt aaccttgtgc tatatgccga tttaggaagg ggggtagatt gagtcaagta    420
   gtaataatat agataactta taagttgttg agaagcagga gagcatctgg gttactcaca    480
   agttttttta aaactttaac gaaagcactt tcggtaatgc ttatgaattt agctatttga    540
   ttcaattact ttaaaaatat ttaggaggta at atg atg tta aga aag aaa aca      593
                                       Met Met Leu Arg Lys Lys Thr
                                                       -25
   aag cag ttg att tct tcc att ctt att tta gtt tta ctt cta tct tta      641
   Lys Gln Leu Ile Ser Ser Ile Leu Ile Leu Val Leu Leu Leu Ser Leu
           -20                 -15                 -10
   ttt ccg gca gct ctt gca gca gaa gga aac act cgt gaa gac aat ttt      689
   Phe Pro Ala Ala Leu Ala Ala Glu Gly Asn Thr Arg Glu Asp Asn Phe
       -5              -1  1               5                   10
   aaa cat tta tta ggt aat gac aat gtt aaa cgc cct tct gag gct ggc      737
   Lys His Leu Leu Gly Asn Asp Asn Val Lys Arg Pro Ser Glu Ala Gly
                           15                  20                  25
   gca tta caa tta caa gaa gtc gat gga caa atg aca tta gta gat caa          785
   Ala Leu Gln Leu Gln Glu Val Asp Gly Gln Met Thr Leu Val Asp Gln
               30                  35                  40
   cat gga gaa aaa att caa tta cgt gga atg agt aca cac gga tta cag          833
   His Gly Glu Lys Ile Gln Leu Arg Gly Met Ser Thr His Gly Leu Gln
           45                  50                  55
   tgg ttt cct gag atc ttg aat gat aac gca tac aaa gct ctt tct aac          881
   Trp Phe Pro Glu Ile Leu Asn Asp Asn Ala Tyr Lys Ala Leu Ser Asn
       60                  65                  70
   gat tgg gat tcc aat atg att cgt ctt gct atg tat gta ggt gaa aat          929
   Asp Trp Asp Ser Asn Met Ile Arg Leu Ala Met Tyr Val Gly Glu Asn
   75                  80                  85                  90
   ggg tac gct aca aac cct gag tta atc aaa caa aga gtg att gat gga          977
   Gly Tyr Ala Thr Asn Pro Glu Leu Ile Lys Gln Arg Val Ile Asp Gly
                   95                  100                 105
   att gag tta gcg att gaa aat gac atg tat gtt att gtt gac tgg cat          1025
   Ile Glu Leu Ala Ile Glu Asn Asp Met Tyr Val Ile Val Asp Trp His
               110                 115                 120
   gtt cat gcg cca ggt gat cct aga gat cct gtt tat gca ggt gct aaa          1073
   Val His Ala Pro Gly Asp Pro Arg Asp Pro Val Tyr Ala Gly Ala Lys
           125                 130                 135
   gat ttc ttt aga gaa att gca gct tta tac cct aat aat cca cac att          1121
   Asp Phe Phe Arg Glu Ile Ala Ala Leu Tyr Pro Asn Asn Pro His Ile
       140                 145                 150
   att tat gag tta gcg aat gag ccg agt agt aat aat aat ggt gga gca          1169
   Ile Tyr Glu Leu Ala Asn Glu Pro Ser Ser Asn Asn Asn Gly Gly Ala
   155                 160                 165                 170
   ggg att ccg aat aac gaa gaa ggt tgg aaa gcg gta aaa gaa tat gct          1217
   Gly Ile Pro Asn Asn Glu Glu Gly Trp Lys Ala Val Lys Glu Tyr Ala
                   175                 180                 185
   gat cca att gta gaa atg tta cgt aaa agc ggt aat gca gat gac aac          1265
   Asp Pro Ile Val Glu Met Leu Arg Lys Ser Gly Asn Ala Asp Asp Asn
               190                 195                 200
   att atc att gtt ggt agt cca aac tgg agt cag cgt ccg gac tta gca          1313
   Ile Ile Ile Val Gly Ser Pro Asn Trp Ser Gln Arg Pro Asp Leu Ala
           205                 210                 215
   gct gat aat cca att gat gat cac cat aca atg tat act gtt cac ttc          1361
   Ala Asp Asn Pro Ile Asp Asp His His Thr Met Tyr Thr Val His Phe
       220                 225                 230
   tac act ggt tca cat gct gct tca act gaa agc tat ccg tct gaa act          1409
   Tyr Thr Gly Ser His Ala Ala Ser Thr Glu Ser Tyr Pro Ser Glu Thr
   235                 240                 245                 250
   cct aac tct gaa aga gga aac gta atg agt aac act cgt tat gcg tta          1457
   Pro Asn Ser Glu Arg Gly Asn Val Met Ser Asn Thr Arg Tyr Ala Leu
                   255                 260                 265
   gaa aac gga gta gcg gta ttt gca aca gag tgg gga acg agt caa gct          1505
   Glu Asn Gly Val Ala Val Phe Ala Thr Glu Trp Gly Thr Ser Gln Ala
               270                 275                 280
   agt gga gac ggt ggt cct tac ttt gat gaa gca gat gta tgg att gaa          1553
   Ser Gly Asp Gly Gly Pro Tyr Phe Asp Glu Ala Asp Val Trp Ile Glu
           285                     290                     295
   ttt tta aat gaa aac aac att agc tgg gct aac tgg tct tta acg aat      1601
   Phe Leu Asn Glu Asn Asn Ile Ser Trp Ala Asn Trp Ser Leu Thr Asn
       300                 305                 310
   aaa aat gaa gta tct ggt gca ttt aca cca ttc gag tta ggt aag tct      1649
   Lys Asn Glu Val Ser Gly Ala Phe Thr Pro Phe Glu Leu Gly Lys Ser
   315                 320                 325                 330
   aac gca acc aat ctt gac cca ggt cca gat cat gtg tgg gca cca gaa      1697
   Asn Ala Thr Asn Leu Asp Pro Gly Pro Asp His Val Trp Ala Pro Glu
                   335                 340                 345
   gaa tta agt ctt tct gga gaa tat gta cgt gct cgt att aaa ggt gtg      1745
   Glu Leu Ser Leu Ser Gly Glu Tyr Val Arg Ala Arg Ile Lys Gly Val
               350                 355                 360
   aac tat gag cca atc gac cgt aca aaa tac acg aaa gta ctt tgg gac      1793
   Asn Tyr Glu Pro Ile Asp Arg Thr Lys Tyr Thr Lys Val Leu Trp Asp
           365                 370                 375
   ttt aat gat gga acg aag caa gga ttt gga gtg aat tcg gat tct cca      1841
   Phe Asn Asp Gly Thr Lys Gln Gly Phe Gly Val Asn Ser Asp Ser Pro
       380                 385                 390
   aat aaa gaa ctt att gca gtt gat aat gaa aac aac act ttg aaa gtt      1889
   Asn Lys Glu Leu Ile Ala Val Asp Asn Glu Asn Asn Thr Leu Lys Val
   395                 400                 405                 410
   tcg gga tta gat gta agt aac gat gtt tca gat ggc aac ttc tgg gct      1937
   Ser Gly Leu Asp Val Ser Asn Asp Val Ser Asp Gly Asn Phe Trp Ala
                   415                 420                 425
   aat gct cgt ctt tct gcc aac ggt tgg gga aaa agt gtt gat att tta      1985
   Asn Ala Arg Leu Ser Ala Asn Gly Trp Gly Lys Ser Val Asp Ile Leu
               430                 435                 440
   ggt gct gag aag ctt aca atg gat gtt att gtt gat gaa cca acg acg      2033
   Gly Ala Glu Lys Leu Thr Met Asp Val Ile Val Asp Glu Pro Thr Thr
           445                 450                 455
   gta gct att gcg gcg att cca caa agt agt aaa agt gga tgg gca aat      2081
   Val Ala Ile Ala Ala Ile Pro Gln Ser Ser Lys Ser Gly Trp Ala Asn
       460                 465                 470
   cca gag cgt gct gtt cga gtg aac gcg gaa gat ttt gtc cag caa acg      2129
   Pro Glu Arg Ala Val Arg Val Asn Ala Glu Asp Phe Val Gln Gln Thr
   475                 480                 485                 490
   gac ggt aag tat aaa gct gga tta aca att aca gga gaa gat gct cct      2177
   Asp Gly Lys Tyr Lys Ala Gly Leu Thr Ile Thr Gly Glu Asp Ala Pro
                   495                 500                 505
   aac cta aaa aat atc gct ttt cat gaa gaa gat aac aat atg aac aac      2225
   Asn Leu Lys Asn Ile Ala Phe His Glu Glu Asp Asn Asn Met Asn Asn
               510                 515                 520
   atc att ctg ttc gtg gga act gat gca gct gac gtt att tac tta gat      2273
   Ile Ile Leu Phe Val Gly Thr Asp Ala Ala Asp Val Ile Tyr Leu Asp
           525                 530                 535
   aac att aaa gta att gga aca gaa gtt gaa att cca gtt gtt cat gat      2321
   Asn Ile Lys Val Ile Gly Thr Glu Val Glu Ile Pro Val Val His Asp
       540                 545                 550
   cca aaa gga gaa gct gtt ctt cct tct gtt ttt gaa gac ggt aca cgt      2369
   Pro Lys Gly Glu Ala Val Leu Pro Ser Val Phe Glu Asp Gly Thr Arg
   555                 560                 565                 570
   caa ggt tgg gac tgg gct gga gag tct ggt gtg aaa aca gct tta aca      2417
   Gln Gly Trp Asp Trp Ala Gly Glu Ser Gly Val Lys Thr Ala Leu Thr
                   575                 580                 585
   att gaa gaa gca aac ggt tct aac gcg tta tca tgg gaa ttt gga tat      2465
   Ile Glu Glu Ala Asn Gly Ser Asn Ala Leu Ser Trp Glu Phe Gly Tyr
               590                 595                 600
   cca gaa gta aaa cct agt gat aac tgg gca aca gct cca cgt tta gat      2513
   Pro Glu Val Lys Pro Ser Asp Asn Trp Ala Thr Ala Pro Arg Leu Asp
           605                 610                 615
   ttc tgg aaa tct gac ttg gtt cgc ggt gag aat gat tat gta gct ttt      2561
   Phe Trp Lys Ser Asp Leu Val Arg Gly Glu Asn Asp Tyr Val Ala Phe
       620                 625                 630
   gat ttc tat cta gat cca gtt cgt gca aca gaa ggc gca atg aat atc      2609
   Asp Phe Tyr Leu Asp Pro Val Arg Ala Thr Glu Gly Ala Met Asn Ile
   635                 640                 645                 650
   aat tta gta ttc cag cca cct act aac ggg tat tgg gta caa gca cca      2657
   Asn Leu Val Phe Gln Pro Pro Thr Asn Gly Tyr Trp Val Gln Ala Pro
                   655                 660                 665
   aaa acg tat acg att aac ttt gat gaa tta gag gaa gcg aat caa gta      2705
   Lys Thr Tyr Thr Ile Asn Phe Asp Glu Leu Glu Glu Ala Asn Gln Val
               670                 675                 680
   aat ggt tta tat cac tat gaa gtg aaa att aac gta aga gat att aca      2753
   Asn Gly Leu Tyr His Tyr Glu Val Lys Ile Asn Val Arg Asp Ile Thr
           685                 690                 695
   aac att caa gat gac acg tta cta cgt aac atg atg atc att ttt gca      2801
   Asn Ile Gln Asp Asp Thr Leu Leu Arg Asn Met Met Ile Ile Phe Ala
       700                 705                 710
   gat gta gaa agt gac ttt gca ggg aga gtc ttt gta gat aat gtt cgt      2849
   Asp Val Glu Ser Asp Phe Ala Gly Arg Val Phe Val Asp Asn Val Arg
   715                 720                 725                 730
   ttt gag ggg gct gct act act gag ccg gtt gaa cca gag cca gtt gat      2897
   Phe Glu Gly Ala Ala Thr Thr Glu Pro Val Glu Pro Glu Pro Val Asp
                   735                 740                 745
   cct ggc gaa gag acg cca cct gtc gat gag aag gaa gcg aaa aaa gaa      2945
   Pro Gly Glu Glu Thr Pro Pro Val Asp Glu Lys Glu Ala Lys Lys Glu
               750                 755                 760
   caa aaa gaa gca gag aaa gaa gag aaa gaa gca gta aaa gaa gaa aag      2993
   Gln Lys Glu Ala Glu Lys Glu Glu Lys Glu Ala Val Lys Glu Glu Lys
           765                 770                 775
   aaa gaa gct aaa gaa gaa aag aaa gca gtc aaa aat gag gct aag aaa      3041
   Lys Glu Ala Lys Glu Glu Lys Lys Ala Val Lys Asn Glu Ala Lys Lys
       780                 785                 790
   aaa taatctatta aactagttat agggttatct aaaggtctga tgtagatctt           3094
   Lys
   795
   ttagataacc tttttcttgc ataactggac acagagttgt tattaaagaa agtaag        3150
<210> 2
   <211> 824
   <212> PRT
   <213> Bacillus sp. KSM-S237
<400> 2
   Met Met Leu Arg Lys Lys Thr Lys Gln Leu Ile Ser Ser Ile Leu Ile
                   -25                 -20                 -15
   Leu Val Leu Leu Leu Ser Leu Phe Pro Ala Ala Leu Ala Ala Glu Gly
               -10                 -5                  -1  1
   Asn Thr Arg Glu Asp Asn Phe Lys His Leu Leu Gly Asn Asp Asn Val
       5                   10                  15
   Lys Arg Pro Ser Glu Ala Gly Ala Leu Gln Leu Gln Glu Val Asp Gly
   20                  25                  30                  35
   Gln Met Thr Leu Val Asp Gln His Gly Glu Lys Ile Gln Leu Arg Gly
                   40                  45                  50
   Met Ser Thr His Gly Leu Gln Trp Phe Pro Glu Ile Leu Asn Asp Asn
               55                  60                  65
   Ala Tyr Lys Ala Leu Ser Asn Asp Trp Asp Ser Asn Met Ile Arg Leu
           70                  75                  80
   Ala Met Tyr Val Gly Glu Asn Gly Tyr Ala Thr Asn Pro Glu Leu Ile
       85                  90                  95
   Lys Gln Arg Val Ile Asp Gly Ile Glu Leu Ala Ile Glu Asn Asp Met
   100                 105                 110                 115
   Tyr Val Ile Val Asp Trp His Val His Ala Pro Gly Asp Pro Arg Asp
                   120                 125                 130
   Pro Val Tyr Ala Gly Ala Lys Asp Phe Phe Arg Glu Ile Ala Ala Leu
               135                 140                 145
   Tyr Pro Asn Asn Pro His Ile Ile Tyr Glu Leu Ala Asn Glu Pro Ser
           150                 155                 160
   Ser Asn Asn Asn Gly Gly Ala Gly Ile Pro Asn Asn Glu Glu Gly Trp
       165                 170                 175
   Lys Ala Val Lys Glu Tyr Ala Asp Pro Ile Val Glu Met Leu Arg Lys
   180                 185                 190                 195
   Ser Gly Asn Ala Asp Asp Asn Ile Ile Ile Val Gly Ser Pro Asn Trp
                   200                 205                 210
   Ser Gln Arg Pro Asp Leu Ala Ala Asp Asn Pro Ile Asp Asp His His
               215                 220                 225
   Thr Met Tyr Thr Val His Phe Tyr Thr Gly Ser His Ala Ala Ser Thr
   230 235 240
   Glu Ser Tyr Pro Ser Glu Thr Pro Asn Ser Glu Arg Gly Asn Val Met
   245 250 255
   Ser Asn Thr Arg Tyr Ala Leu Glu Asn Gly Val Ala Val Phe Ala Thr
   260 265 270 275
   Glu Trp Gly Thr Ser Gln Ala Ser Gly Asp Gly Gly Pro Tyr Phe Asp
   280 285 290
   Glu Ala Asp Val Trp Ile Glu Phe Leu Asn Glu Asn Asn Ile Ser Trp
   295 300 305
   Ala Asn Trp Ser Leu Thr Asn Lys Asn Glu Val Ser Gly Ala Phe Thr
   310 315 320
   Pro Phe Glu Leu Gly Lys Ser Asn Ala Thr Asn Leu Asp Pro Gly Pro
   325 330 335
   Asp His Val Trp Ala Pro Glu Glu Leu Ser Leu Ser Gly Glu Tyr Val
   340 345 350 355
   Arg Ala Arg Ile Lys Gly Val Asn Tyr Glu Pro Ile Asp Arg Thr Lys
   360 365 370
   Tyr Thr Lys Val Leu Trp Asp Phe Asn Asp Gly Thr Lys Gln Gly Phe
   375 380 385
   Gly Val Asn Ser Asp Ser Pro Asn Lys Glu Leu Ile Ala Val Asp Asn
   390 395 400
   Glu Asn Asn Thr Leu Lys Val Ser Gly Leu Asp Val Ser Asn Asp Val
   405 410 415
   Ser Asp Gly Asn Phe Trp Ala Asn Ala Arg Leu Ser Ala Asn Gly Trp
   420 425 430 435
   Gly Lys Ser Val Asp Ile Leu Gly Ala Glu Lys Leu Thr Met Asp Val
   440 445 450
   Ile Val Asp Glu Pro Thr Thr Val Ala Ile Ala Ala Ile Pro Gln Ser
   455 460 465
   Ser Lys Ser Gly Trp Ala Asn Pro Glu Arg Ala Val Arg Val Asn Ala
   470 475 480
   Glu Asp Phe Val Gln Gln Thr Asp Gly Lys Tyr Lys Ala Gly Leu Thr
   485 490 495
   Ile Thr Gly Glu Asp Ala Pro Asn Leu Lys Asn Ile Ala Phe His Glu
   500 505 510 515
   Glu Asp Asn Asn Met Asn Asn Ile Ile Leu Phe Val Gly Thr Asp Ala
   520 525 530
   Ala Asp Val Ile Tyr Leu Asp Asn Ile Lys Val Ile Gly Thr Glu Val
   535 540 545
   Glu Ile Pro Val Val His Asp Pro Lys Gly Glu Ala Val Leu Pro Ser
   550 555 560
   Val Phe Glu Asp Gly Thr Arg Gln Gly Trp Asp Trp Ala Gly Glu Ser
   565 570 575
   Gly Val Lys Thr Ala Leu Thr Ile Glu Glu Ala Asn Gly Ser Asn Ala
   580 585 590 595
   Leu Ser Trp Glu Phe Gly Tyr Pro Glu Val Lys Pro Ser Asp Asn Trp
   600 605 610
   Ala Thr Ala Pro Arg Leu Asp Phe Trp Lys Ser Asp Leu Val Arg Gly
   615 620 625
   Glu Asn Asp Tyr Val Ala Phe Asp Phe Tyr Leu Asp Pro Val Arg Ala
   630 635 640
   Thr Glu Gly Ala Met Asn Ile Asn Leu Val Phe Gln Pro Pro Thr Asn
   645 650 655
   Gly Tyr Trp Val Gln Ala Pro Lys Thr Tyr Thr Ile Asn Phe Asp Glu
   660 665 670 675
   Leu Glu Glu Ala Asn Gln Val Asn Gly Leu Tyr His Tyr Glu Val Lys
   680 685 690
   Ile Asn Val Arg Asp Ile Thr Asn Ile Gln Asp Asp Thr Leu Leu Arg
   695 700 705
   Asn Met Met Ile Ile Phe Ala Asp Val Glu Ser Asp Phe Ala Gly Arg
   710 715 720
   Val Phe Val Asp Asn Val Arg Phe Glu Gly Ala Ala Thr Thr Glu Pro
   725 730 735
   Val Glu Pro Glu Pro Val Asp Pro Gly Glu Glu Thr Pro Pro Val Asp
   740 745 750 755
   Glu Lys Glu Ala Lys Lys Glu Gln Lys Glu Ala Glu Lys Glu Glu Lys
   760 765 770
   Glu Ala Val Lys Glu Glu Lys Lys Glu Ala Lys Glu Glu Lys Lys Ala
   775 780 785
   Val Lys Asn Glu Ala Lys Lys Lys
   790 795
<210> 3
   <211> 3332
   <212> DNA
   <213> Bacillus sp. KSM-64
<220>
   <221> CDS
   <222> (610)..(3075)
   <223>
<220>
   <221> sig_peptide
   <222> (610)..(696)
   <223>
<220>
   <221> mat_peptide
   <222> (697)..()
   <223>
<400> 3
   agtacttacc attttagagt caaaagatag aagccaagca ggatttgccg atgcaaccgg      60
   cttatattta gagggaattt ctttttaaat tgaatacgga ataaaatcag gtaaacaggt     120
   cctgatttta tttttttgaa tttttttgag aactaaagat tgaaatagaa gtagaagaca      180
   acggacataa gaaaattgta ttagttttaa ttatagaaaa cgcttttcta taattattta     240
   tacctagaac gaaaatactg tttcgaaagc ggtttactat aaaaccttat attccggctc     300
   tttttttaaa cagggggtga aaattcactc tagtattcta atttcaacat gctataataa       360
   atttgtaaga cgcaatatac atcttttttt tatgatattt gtaagcggtt aaccttgtgc     420
   tatatgccga tttaggaagg gggtagattg agtcaagtag tcataattta gataacttat     480
   aagttgttga gaagcaggag agaatctggg ttactcacaa gttttttaaa acattatcga      540
   aagcactttc ggttatgctt atgaatttag ctatttgatt caattacttt aataatttta     600
   ggaggtaat atg atg tta aga aag aaa aca aag cag ttg att tct tcc att     651
             Met Met Leu Arg Lys Lys Thr Lys Gln Leu Ile Ser Ser Ile
                             -25                 -20
   ctt att tta gtt tta ctt cta tct tta ttt ccg aca gct ctt gca gca       699
   Leu Ile Leu Val Leu Leu Leu Ser Leu Phe Pro Thr Ala Leu Ala Ala
   -15                 -10                 -5                  -1   1
   gaa gga aac act cgt gaa gac aat ttt aaa cat tta tta ggt aat gac       747
   Glu Gly Asn Thr Arg Glu Asp Asn Phe Lys His Leu Leu Gly Asn Asp
               5                   10                  15
   aat gtt aaa cgc cct tct gag gct ggc gca tta caa tta caa gaa gtc       795
   Asn Val Lys Arg Pro Ser Glu Ala Gly Ala Leu Gln Leu Gln Glu Val
           20                  25                  30
   gat gga caa atg aca tta gta gat caa cat gga gaa aaa att caa tta       843
   Asp Gly Gln Met Thr Leu Val Asp Gln His Gly Glu Lys Ile Gln Leu
       35                  40                  45
   cgt gga atg agt aca cac gga tta caa tgg ttt cct gag atc ttg aat      891
   Arg Gly Met Ser Thr His Gly Leu Gln Trp Phe Pro Glu Ile Leu Asn
   50                  55                  60                  65
   gat aac gca tac aaa gct ctt gct aac gat tgg gaa tca aat atg att      939
   Asp Asn Ala Tyr Lys Ala Leu Ala Asn Asp Trp Glu Ser Asn Met Ile
                   70                  75                  80
   cgt cta gct atg tat gtc ggt gaa aat ggc tat gct tca aat cca gag      987
   Arg Leu Ala Met Tyr Val Gly Glu Asn Gly Tyr Ala Ser Asn Pro Glu
               85                  90                  95
   tta att aaa agc aga gtc att aaa gga ata gat ctt gct att gaa aat     1035
   Leu Ile Lys Ser Arg Val Ile Lys Gly Ile Asp Leu Ala Ile Glu Asn
           100                 105                 110
   gac atg tat gtc atc gtt gat tgg cat gta cat gca cct ggt gat cct     1083
   Asp Met Tyr Val Ile Val Asp Trp His Val His Ala Pro Gly Asp Pro
       115                 120                 125
   aga gat ccc gtt tac gct gga gca gaa gat ttc ttt aga gat att gca     1131
   Arg Asp Pro Val Tyr Ala Gly Ala Glu Asp Phe Phe Arg Asp Ile Ala
   130                 135                 140                 145
   gca tta tat cct aac aat cca cac att att tat gag tta gcg aat gag     1179
   Ala Leu Tyr Pro Asn Asn Pro His Ile Ile Tyr Glu Leu Ala Asn Glu
                   150                 155                 160
   cca agt agt aac aat aat ggt gga gct ggg att cca aat aat gaa gaa     1227
   Pro Ser Ser Asn Asn Asn Gly Gly Ala Gly Ile Pro Asn Asn Glu Glu
               165                 170                 175
   ggt tgg aat gcg gta aaa gaa tac gct gat cca att gta gaa atg tta     1275
   Gly Trp Asn Ala Val Lys Glu Tyr Ala Asp Pro Ile Val Glu Met Leu
           180                 185                 190
   cgt gat agc ggg aac gca gat gac aat att atc att gtg ggt agt cca     1323
   Arg Asp Ser Gly Asn Ala Asp Asp Asn Ile Ile Ile Val Gly Ser Pro
       195                 200                 205
   aac tgg agt cag cgt cct gac tta gca gct gat aat cca att gat gat     1371
   Asn Trp Ser Gln Arg Pro Asp Leu Ala Ala Asp Asn Pro Ile Asp Asp
   210                 215                 220                 225
   cac cat aca atg tat act gtt cac ttc tac act ggt tca cat gct gct     1419
   His His Thr Met Tyr Thr Val His Phe Tyr Thr Gly Ser His Ala Ala
                   230                 235                 240
   tca act gaa agc tat ccg cct gaa act cct aac tct gaa aga gga aac     1467
   Ser Thr Glu Ser Tyr Pro Pro Glu Thr Pro Asn Ser Glu Arg Gly Asn
               245                 250                 255
   gta atg agt aac act cgt tat gcg tta gaa aac gga gta gca gta ttt     1515
   Val Met Ser Asn Thr Arg Tyr Ala Leu Glu Asn Gly Val Ala Val Phe
           260                 265                 270
   gca aca gag tgg gga act agc caa gca aat gga gat ggt ggt cct tac     1563
   Ala Thr Glu Trp Gly Thr Ser Gln Ala Asn Gly Asp Gly Gly Pro Tyr
       275                 280                 285
   ttt gat gaa gca gat gta tgg att gag ttt tta aat gaa aac aac att     1611
   Phe Asp Glu Ala Asp Val Trp Ile Glu Phe Leu Asn Glu Asn Asn Ile
   290                 295                 300                 305
   agc tgg gct aac tgg tct tta acg aat aaa aat gaa gta tct ggt gca     1659
   Ser Trp Ala Asn Trp Ser Leu Thr Asn Lys Asn Glu Val Ser Gly Ala
                   310                 315                 320
   ttt aca cca ttc gag tta ggt aag tct aac gca aca agt ctt gac cca      1707
   Phe Thr Pro Phe Glu Leu Gly Lys Ser Asn Ala Thr Ser Leu Asp Pro
               325                 330                 335
   ggg cca gac caa gta tgg gta cca gaa gag tta agt ctt tct gga gaa      1755
   Gly Pro Asp Gln Val Trp Val Pro Glu Glu Leu Ser Leu Ser Gly Glu
           340                 345                 350
   tat gta cgt gct cgt att aaa ggt gtg aac tat gag cca atc gac cgt      1803
   Tyr Val Arg Ala Arg Ile Lys Gly Val Asn Tyr Glu Pro Ile Asp Arg
       355                 360                 365
   aca aaa tac acg aaa gta ctt tgg gac ttt aat gat gga acg aag caa      1851
   Thr Lys Tyr Thr Lys Val Leu Trp Asp Phe Asn Asp Gly Thr Lys Gln
   370                 375                 380                 385
   gga ttt gga gtg aat gga gat tct cca gtt gaa gat gta gtt att gag      1899
   Gly Phe Gly Val Asn Gly Asp Ser Pro Val Glu Asp Val Val Ile Glu
                   390                 395                 400
   aat gaa gcg ggc gct tta aaa ctt tca gga tta gat gca agt aat gat      1947
   Asn Glu Ala Gly Ala Leu Lys Leu Ser Gly Leu Asp Ala Ser Asn Asp
               405                 410                 415
   gtt tct gaa ggt aat tac tgg gct aat gct cgt ctt tct gcc gac ggt      1995
   Val Ser Glu Gly Asn Tyr Trp Ala Asn Ala Arg Leu Ser Ala Asp Gly
           420                 425                 430
   tgg gga aaa agt gtt gat att tta ggt gct gaa aaa ctt act atg gat      2043
   Trp Gly Lys Ser Val Asp Ile Leu Gly Ala Glu Lys Leu Thr Met Asp
       435                 440                 445
   gtg att gtt gat gag ccg acc acg gta tca att gct gca att cca caa      2091
   Val Ile Val Asp Glu Pro Thr Thr Val Ser Ile Ala Ala Ile Pro Gln
   450                 455                 460                 465
   ggg cca tca gcc aat tgg gtt aat cca aat cgt gca att aag gtt gag      2139
   Gly Pro Ser Ala Asn Trp Val Asn Pro Asn Arg Ala Ile Lys Val Glu
                   470                 475                 480
   cca act aat ttc gta ccg tta gga gat aag ttt aaa gcg gaa tta act      2187
   Pro Thr Asn Phe Val Pro Leu Gly Asp Lys Phe Lys Ala Glu Leu Thr
               485                 490                 495
   ata act tca gct gac tct cca tcg tta gaa gct att gcg atg cat gct      2235
   Ile Thr Ser Ala Asp Ser Pro Ser Leu Glu Ala Ile Ala Met His Ala
           500                 505                 510
   gaa aat aac aac atc aac aac atc att ctt ttt gta gga act gaa ggt      2283
   Glu Asn Asn Asn Ile Asn Asn Ile Ile Leu Phe Val Gly Thr Glu Gly
       515                 520                 525
   gct gat gtt atc tat tta gat aac att aaa gta att gga aca gaa gtt      2331
   Ala Asp Val Ile Tyr Leu Asp Asn Ile Lys Val Ile Gly Thr Glu Val
   530                 535                 540                 545
   gaa att cca gtt gtt cat gat cca aaa gga gaa gct gtt ctt cct tct      2379
   Glu Ile Pro Val Val His Asp Pro Lys Gly Glu Ala Val Leu Pro Ser
                   550                 555                 560
   gtt ttt gaa gac ggt aca cgt caa ggt tgg gac tgg gct gga gag tct      2427
   Val Phe Glu Asp Gly Thr Arg Gln Gly Trp Asp Trp Ala Gly Glu Ser
               565                 570                 575
   ggt gtg aaa aca gct tta aca att gaa gaa gca aac ggt tct aac gcg      2475
   Gly Val Lys Thr Ala Leu Thr Ile Glu Glu Ala Asn Gly Ser Asn Ala
           580                 585                 590
   tta tca tgg gaa ttt gga tac cca gaa gta aaa cct agt gat aac tgg      2523
   Leu Ser Trp Glu Phe Gly Tyr Pro Glu Val Lys Pro Ser Asp Asn Trp
       595                 600                 605
   gca aca gct cca cgt tta gat ttc tgg aaa tct gac ttg gtt cgc ggt      2571
   Ala Thr Ala Pro Arg Leu Asp Phe Trp Lys Ser Asp Leu Val Arg Gly
   610                 615                 620                 625
   gaa aat gat tat gta act ttt gat ttc tat cta gat cca gtt cgt gca      2619
   Glu Asn Asp Tyr Val Thr Phe Asp Phe Tyr Leu Asp Pro Val Arg Ala
                   630                 635                 640
   aca gaa ggc gca atg aat atc aat tta gta ttc cag cca cct act aac      2667
   Thr Glu Gly Ala Met Asn Ile Asn Leu Val Phe Gln Pro Pro Thr Asn
               645                 650                 655
   ggg tat tgg gta caa gca cca aaa acg tat acg att aac ttt gat gaa      2715
   Gly Tyr Trp Val Gln Ala Pro Lys Thr Tyr Thr Ile Asn Phe Asp Glu
           660                 665                 670
   tta gag gaa gcg aat caa gta aat ggt tta tat cac tat gaa gtg aaa      2763
   Leu Glu Glu Ala Asn Gln Val Asn Gly Leu Tyr His Tyr Glu Val Lys
       675                 680                 685
   att aac gta aga gat att aca aac att caa gat gac acg tta cta cgt      2811
   Ile Asn Val Arg Asp Ile Thr Asn Ile Gln Asp Asp Thr Leu Leu Arg
   690                 695                 700                 705
   aac atg atg atc att ttt gca gat gta gaa agt gac ttt gca ggg aga      2859
   Asn Met Met Ile Ile Phe Ala Asp Val Glu Ser Asp Phe Ala Gly Arg
                   710                 715                 720
   gtc ttt gta gat aat gtt cgt ttt gag ggg gct gct act act gag ccg      2907
   Val Phe Val Asp Asn Val Arg Phe Glu Gly Ala Ala Thr Thr Glu Pro
               725                 730                 735
   gtt gaa cca gag cca gtt gat cct ggc gaa gag acg ccg cct gtc gat      2955
   Val Glu Pro Glu Pro Val Asp Pro Gly Glu Glu Thr Pro Pro Val Asp
           740                 745                 750
   gag aag gaa gcg aaa aaa gaa caa aaa gaa gca gag aaa gaa gag aaa      3003
   Glu Lys Glu Ala Lys Lys Glu Gln Lys Glu Ala Glu Lys Glu Glu Lys
       755                 760                 765
   gaa gca gta aaa gaa gaa aag aaa gaa gct aaa gaa gaa aag aaa gca      3051
   Glu Ala Val Lys Glu Glu Lys Lys Glu Ala Lys Glu Glu Lys Lys Ala
   770                 775                 780                 785
   atc aaa aat gag gct acg aaa aaa taatctaata aactagttat agggttatct     3105
   Ile Lys Asn Glu Ala Thr Lys Lys
                   790
   aaaggtctga tgcagatctt ttagataacc tttttttgca taactggaca tagaatggtt     3165
   attaaagaaa gcaaggtgtt tatacgatat taaaaaggta gcgattttaa attgaaacct    3225
   ttaataatgt cttgtgatag aatgatgaag taatttaaga gggggaaacg aagtgaaaac    3285
   ggaaatttct agtagaagaa aaacagacca agaaatactg caagctt                  3332
<210> 4
   <211> 822
   <212> PRT
   <213> Bacillus sp. KSM-64
<400> 4
   Met Met Leu Arg Lys Lys Thr Lys Gln Leu Ile Ser Ser Ile Leu Ile
                   -25                 -20                 -15
   Leu Val Leu Leu Leu Ser Leu Phe Pro Thr Ala Leu Ala Ala Glu Gly
               -10                 -5              -1  1
   Asn Thr Arg Glu Asp Asn Phe Lys His Leu Leu Gly Asn Asp Asn Val
       5                   10                  15
   Lys Arg Pro Ser Glu Ala Gly Ala Leu Gln Leu Gln Glu Val Asp Gly
   20                  25                  30                  35
   Gln Met Thr Leu Val Asp Gln His Gly Glu Lys Ile Gln Leu Arg Gly
                   40                  45                  50
   Met Ser Thr His Gly Leu Gln Trp Phe Pro Glu Ile Leu Asn Asp Asn
               55                  60                  65
   Ala Tyr Lys Ala Leu Ala Asn Asp Trp Glu Ser Asn Met Ile Arg Leu
           70                  75                  80
   Ala Met Tyr Val Gly Glu Asn Gly Tyr Ala Ser Asn Pro Glu Leu Ile
       85                  90                  95
   Lys Ser Arg Val Ile Lys Gly Ile Asp Leu Ala Ile Glu Asn Asp Met
   100                 105                 110                 115
   Tyr Val Ile Val Asp Trp His Val His Ala Pro Gly Asp Pro Arg Asp
                   120                 125                 130
   Pro Val Tyr Ala Gly Ala Glu Asp Phe Phe Arg Asp Ile Ala Ala Leu
               135                 140                 145
   Tyr Pro Asn Asn Pro His Ile Ile Tyr Glu Leu Ala Asn Glu Pro Ser
           150                 155                 160
   Ser Asn Asn Asn Gly Gly Ala Gly Ile Pro Asn Asn Glu Glu Gly Trp
       165                 170                 175
   Asn Ala Val Lys Glu Tyr Ala Asp Pro Ile Val Glu Met Leu Arg Asp
   180                 185                 190                 195
   Ser Gly Asn Ala Asp Asp Asn Ile Ile Ile Val Gly Ser Pro Asn Trp
                   200                 205                 210
   Ser Gln Arg Pro Asp Leu Ala Ala Asp Asn Pro Ile Asp Asp His His
               215                 220                 225
   Thr Met Tyr Thr Val His Phe Tyr Thr Gly Ser His Ala Ala Ser Thr
           230                 235                 240
   Glu Ser Tyr Pro Pro Glu Thr Pro Asn Ser Glu Arg Gly Asn Val Met
       245                 250                 255
   Ser Asn Thr Arg Tyr Ala Leu Glu Asn Gly Val Ala Val Phe Ala Thr
   260                 265                 270                 275
   Glu Trp Gly Thr Ser Gln Ala Asn Gly Asp Gly Gly Pro Tyr Phe Asp
                   280                 285                 290
   Glu Ala Asp Val Trp Ile Glu Phe Leu Asn Glu Asn Asn Ile Ser Trp
               295                 300                 305
   Ala Asn Trp Ser Leu Thr Asn Lys Asn Glu Val Ser Gly Ala Phe Thr
           310                 315                 320
   Pro Phe Glu Leu Gly Lys Ser Asn Ala Thr Ser Leu Asp Pro Gly Pro
       325                 330                 335
   Asp Gln Val Trp Val Pro Glu Glu Leu Ser Leu Ser Gly Glu Tyr Val
   340                 345                 350                 355
   Arg Ala Arg Ile Lys Gly Val Asn Tyr Glu Pro Ile Asp Arg Thr Lys
                   360                 365                 370
   Tyr Thr Lys Val Leu Trp Asp Phe Asn Asp Gly Thr Lys Gln Gly Phe
               375                 380                 385
   Gly Val Asn Gly Asp Ser Pro Val Glu Asp Val Val Ile Glu Asn Glu
           390                 395                 400
   Ala Gly Ala Leu Lys Leu Ser Gly Leu Asp Ala Ser Asn Asp Val Ser
       405                 410                 415
   Glu Gly Asn Tyr Trp Ala Asn Ala Arg Leu Ser Ala Asp Gly Trp Gly
   420                 425                 430                 435
   Lys Ser Val Asp Ile Leu Gly Ala Glu Lys Leu Thr Met Asp Val Ile
                   440                 445                 450
   Val Asp Glu Pro Thr Thr Val Ser Ile Ala Ala Ile Pro Gln Gly Pro
               455                 460                 465
   Ser Ala Asn Trp Val Asn Pro Asn Arg Ala Ile Lys Val Glu Pro Thr
           470                 475                 480
   Asn Phe Val Pro Leu Gly Asp Lys Phe Lys Ala Glu Leu Thr Ile Thr
       485                 490                 495
   Ser Ala Asp Ser Pro Ser Leu Glu Ala Ile Ala Met His Ala Glu Asn
   500                 505                 510                 515
   Asn Asn Ile Asn Asn Ile Ile Leu Phe Val Gly Thr Glu Gly Ala Asp
   520 525 530
   Val Ile Tyr Leu Asp Asn Ile Lys Val Ile Gly Thr Glu Val Glu Ile
   535 540 545
   Pro Val Val His Asp Pro Lys Gly Glu Ala Val Leu Pro Ser Val Phe
   550 555 560
   Glu Asp Gly Thr Arg Gln Gly Trp Asp Trp Ala Gly Glu Ser Gly Val
   565 570 575
   Lys Thr Ala Leu Thr Ile Glu Glu Ala Asn Gly Ser Asn Ala Leu Ser
   580 585 590 595
   Trp Glu Phe Gly Tyr Pro Glu Val Lys Pro Ser Asp Asn Trp Ala Thr
   600 605 610
   Ala Pro Arg Leu Asp Phe Trp Lys Ser Asp Leu Val Arg Gly Glu Asn
   615 620 625
   Asp Tyr Val Thr Phe Asp Phe Tyr Leu Asp Pro Val Arg Ala Thr Glu
   630 635 640
   Gly Ala Met Asn Ile Asn Leu Val Phe Gln Pro Pro Thr Asn Gly Tyr
   645 650 655
   Trp Val Gln Ala Pro Lys Thr Tyr Thr Ile Asn Phe Asp Glu Leu Glu
   660 665 670 675
   Glu Ala Asn Gln Val Asn Gly Leu Tyr His Tyr Glu Val Lys Ile Asn
   680 685 690
   Val Arg Asp Ile Thr Asn Ile Gln Asp Asp Thr Leu Leu Arg Asn Met
   695 700 705
   Met Ile Ile Phe Ala Asp Val Glu Ser Asp Phe Ala Gly Arg Val Phe
   710 715 720
   Val Asp Asn Val Arg Phe Glu Gly Ala Ala Thr Thr Glu Pro Val Glu
   725 730 735
   Pro Glu Pro Val Asp Pro Gly Glu Glu Thr Pro Pro Val Asp Glu Lys
   740 745 750 755
   Glu Ala Lys Lys Glu Gln Lys Glu Ala Glu Lys Glu Glu Lys Glu Ala
   760 765 770
   Val Lys Glu Glu Lys Lys Glu Ala Lys Glu Glu Lys Lys Ala Ile Lys
   775 780 785
   Asn Glu Ala Thr Lys Lys
   790
<210> 5
   <211> 2343
   <212> DNA
   <213> Bacillus sp.pHSP-K38
<220>
   <221> CDS
   <222> (580)..(2067)
   <223>
<220>
   <221> sig_peptide
   <222> (580)..(627)
   <223>
<220>
   <221> mat_peptide
   <222> (628)..()
   <223>
<400> 5
   agatctagca ggatttgccg atgcaaccgg cttatattta gagggaattt ctttttaaat      60
   tgaatacgga ataaaatcag gtaaacaggt cctgatttta tttttttgaa tttttttgag     120
   aactaaagat tgaaatagaa gtagaagaca acggacataa gaaaattgta ttagttttaa     180
   ttatagaaaa cgcttttcta taattattta tacctagaac gaaaatactg tttcgaaagc     240
   ggtttactat aaaaccttat attccggctc tttttttaaa cagggggtga aaattcactc     300
   tagtattcta atttcaacat gctataataa atttgtaaga cgcaatatac atcttttttt     360
   tatgatattt gtaagcggtt aaccttgtgc tatatgccga tttaggaagg gggtagattg     420
   agtcaagtag tcataattta gataacttat aagttgttga gaagcaggag agaatctggg     480
   ttactcacaa gttttttaaa acattatcga aagcactttc ggttatgctt atgaatttag     540
   ctatttgatt caattacttt aataatttta ggaggtaat atg atg tta aga aag        594
                                              Met Met Leu Arg Lys
                                                  -15
   aaa aca aag cag ttg ggt cga cca gca caa gcc gat gga ttg aac ggt       642
   Lys Thr Lys Gln Leu Gly Arg Pro Ala Gln Ala Asp Gly Leu Asn Gly
       -10                 -5                  -1  1               5
   acg atg atg cag tat tat gag tgg cat ttg gaa aac gac ggg cag cat       690
   Thr Met Met Gln Tyr Tyr Glu Trp His Leu Glu Asn Asp Gly Gln His
                   10                  15                  20
   tgg aat cgg ttg cac gat gat gcc gca gct ttg agt gat gct ggt att       738
   Trp Asn Arg Leu His Asp Asp Ala Ala Ala Leu Ser Asp Ala Gly Ile
               25                  30                  35
   aca gct att tgg att ccg cca gcc tac aaa ggt aat agt cag gcg gat       786
   Thr Ala Ile Trp Ile Pro Pro Ala Tyr Lys Gly Asn Ser Gln Ala Asp
           40                  45                  50
   gtt ggg tac ggt gca tac gat ctt tat gat tta gga gag ttc aat caa       834
   Val Gly Tyr Gly Ala Tyr Asp Leu Tyr Asp Leu Gly Glu Phe Asn Gln
       55                  60                  65
   aag ggt act gtt cga acg aaa tac gga act aag gca cag ctt gaa cga       882
   Lys Gly Thr Val Arg Thr Lys Tyr Gly Thr Lys Ala Gln Leu Glu Arg
   70                  75                  80                  85
   gct att ggg tcc ctt aaa tct aat gat atc aat gta tac gga gat gtc       930
   Ala Ile Gly Ser Leu Lys Ser Asn Asp Ile Asn Val Tyr Gly Asp Val
                   90                  95                  100
   gtg atg aat cat aaa atg gga gct gat ttt acg gag gca gtg caa gct       978
   Val Met Asn His Lys Met Gly Ala Asp Phe Thr Glu Ala Val Gln Ala
               105                 110                 115
   gtt caa gta aat cca acg aat cgt tgg cag gat att tca ggt gcc tac       1026
   Val Gln Val Asn Pro Thr Asn Arg Trp Gln Asp Ile Ser Gly Ala Tyr
           120                 125                 130
   acg att gat gcg tgg acg ggt ttc gac ttt tca ggg cgt aac aac gcc       1074
   Thr Ile Asp Ala Trp Thr Gly Phe Asp Phe Ser Gly Arg Asn Asn Ala
       135                 140                 145
   tat tca gat ttt aag tgg aga tgg ttc cat ttt aat ggt gtt gac tgg       1122
   Tyr Ser Asp Phe Lys Trp Arg Trp Phe His Phe Asn Gly Val Asp Trp
   150                 155                 160                 165
   gat cag cgc tat caa gaa aat cat att ttc cgc ttt gca aat acg aac       1170
   Asp Gln Arg Tyr Gln Glu Asn His Ile Phe Arg Phe Ala Asn Thr Asn
                   170                 175                 180
   tgg aac tgg cga gtg gat gaa gag aac ggt aat tat gat tac ctg tta       1218
   Trp Asn Trp Arg Val Asp Glu Glu Asn Gly Asn Tyr Asp Tyr Leu Leu
               185                 190                 195
   gga tcg aat atc gac ttt agt cat cca gaa gta caa gat gag ttg aag       1266
   Gly Ser Asn Ile Asp Phe Ser His Pro Glu Val Gln Asp Glu Leu Lys
           200                 205                 210
   gat tgg ggt agc tgg ttt acc gat gag tta gat ttg gat ggt tat cgt       1314
   Asp Trp Gly Ser Trp Phe Thr Asp Glu Leu Asp Leu Asp Gly Tyr Arg
       215                 220                 225
   tta gat gct att aaa cat att cca ttc tgg tat aca tct gat tgg gtt       1362
   Leu Asp Ala Ile Lys His Ile Pro Phe Trp Tyr Thr Ser Asp Trp Val
   230                 235                 240                 245
   cgg cat cag cgc aac gaa gca gat caa gat tta ttt gtc gta ggg gaa       1410
   Arg His Gln Arg Asn Glu Ala Asp Gln Asp Leu Phe Val Val Gly Glu
                   250                 255                 260
   tat tgg aag gat gac gta ggt gct ctc gaa ttt tat tta gat gaa atg       1458
   Tyr Trp Lys Asp Asp Val Gly Ala Leu Glu Phe Tyr Leu Asp Glu Met
               265                 270                 275
   aat tgg gag atg tct cta ttc gat gtt cca ctt aat tat aat ttt tac       1506
   Asn Trp Glu Met Ser Leu Phe Asp Val Pro Leu Asn Tyr Asn Phe Tyr
           280                 285                 290
   cgg gct tca caa caa ggt gga agc tat gat atg cgt aat att tta cga       1554
   Arg Ala Ser Gln Gln Gly Gly Ser Tyr Asp Met Arg Asn Ile Leu Arg
       295                 300                 305
   gga tct tta gta gaa gcg cat ccg atg cat gca gtt acg ttt gtt gat       1602
   Gly Ser Leu Val Glu Ala His Pro Met His Ala Val Thr Phe Val Asp
   310                 315                 320                 325
   aat cat gat act cag cca ggg gag tca tta gag tca tgg gtt gct gat       1650
   Asn His Asp Thr Gln Pro Gly Glu Ser Leu Glu Ser Trp Val Ala Asp
                   330                 335                 340
   tgg ttt aag cca ctt gct tat gcg aca att ttg acg cgt gaa ggt ggt    1698
   Trp Phe Lys Pro Leu Ala Tyr Ala Thr Ile Leu Thr Arg Glu Gly Gly
               345                 350                 355
   tat cca aat gta ttt tac ggt gat tac tat ggg att cct aac gat aac    1746
   Tyr Pro Asn Val Phe Tyr Gly Asp Tyr Tyr Gly Ile Pro Asn Asp Asn
           360                 365                 370
   att tca gct aaa aaa gat atg att gat gag ctg ctt gat gca cgt caa    1794
   Ile Ser Ala Lys Lys Asp Met Ile Asp Glu Leu Leu Asp Ala Arg Gln
       375                 380                 385
   aat tac gca tat ggc acg cag cat gac tat ttt gat cat tgg gat gtt    1842
   Asn Tyr Ala Tyr Gly Thr Gln His Asp Tyr Phe Asp His Trp Asp Val
   390                 395                 400                     405
   gta gga tgg act agg gaa gga tct tcc tcc aga cct aat tca ggc ctt    1890
   Val Gly Trp Thr Arg Glu Gly Ser Ser Ser Arg Pro Asn Ser Gly Leu
                   410                 415                 420
   gcg act att atg tcg aat gga cct ggt ggt tcc aag tgg atg tat gta    1938
   Ala Thr Ile Met Ser Asn Gly Pro Gly Gly Ser Lys Trp Met Tyr Val
               425                 430                 435
   gga cgt cag aat gca gga caa aca tgg aca gat tta act ggt aat aac    1986
   Gly Arg Gln Asn Ala Gly Gln Thr Trp Thr Asp Leu Thr Gly Asn Asn
           440                 445                 450
   gga gcg tcc gtt aca att aat ggc gat gga tgg ggc gaa ttc ttt acg    2034
   Gly Ala Ser Val Thr Ile Asn Gly Asp Gly Trp Gly Glu Phe Phe Thr
       455                 460                 465
   aat gga gga tct gta tcc gtg tac gtg aac caa taacaaaaag ccttgagaag    2087
   Asn Gly Gly Ser Val Ser Val Tyr Val Asn Gln
   470                 475                 480
   ggattcctcc ctaactcaag gctttcttta tgtcgcttag ctttacgctt ctacgacttt    2147
   gaagcttggg gatccgtcga gacaaggtaa aggataaaac agcacaattc caagaaaaac    2207
   acgatttaga acctaaaaag aacgaatttg aactaactca taaccgagag gtaaaaaaag    2267
   aacgaagtcg agatcaggga atgagtttat aaaataaaaa aagcacctga aaaggtgtct    2327
   ttttttgatg tctaga                                                  2343
<210> 6
   <211> 496
   <212> PRT
   <213> Bacillus sp.pHSP-K38
<400> 6
   Met Met Leu Arg Lys Lys Thr Lys Gln Leu Gly Arg Pro Ala Gln Ala
       -15                 -10                 -5                  -1
   Asp Gly Leu Asn Gly Thr Met Met Gln Tyr Tyr Glu Trp His Leu Glu
   1               5                   10                  15
   Asn Asp Gly Gln His Trp Asn Arg Leu His Asp Asp Ala Ala Ala Leu
               20                  25                  30
   Ser Asp Ala Gly Ile Thr Ala Ile Trp Ile Pro Pro Ala Tyr Lys Gly
           35                  40                  45
   Asn Ser Gln Ala Asp Val Gly Tyr Gly Ala Tyr Asp Leu Tyr Asp Leu
       50                  55                  60
   Gly Glu Phe Asn Gln Lys Gly Thr Val Arg Thr Lys Tyr Gly Thr Lys
   65                  70                  75                  80
   Ala Gln Leu Glu Arg Ala Ile Gly Ser Leu Lys Ser Asn Asp Ile Asn
                   85                  90                  95
   Val Tyr Gly Asp Val Val Met Asn His Lys Met Gly Ala Asp Phe Thr
               100                 105                 110
   Glu Ala Val Gln Ala Val Gln Val Asn Pro Thr Asn Arg Trp Gln Asp
           115                 120                 125
   Ile Ser Gly Ala Tyr Thr Ile Asp Ala Trp Thr Gly Phe Asp Phe Ser
       130                 135                 140
   Gly Arg Asn Asn Ala Tyr Ser Asp Phe Lys Trp Arg Trp Phe His Phe
   145                 150                 155                 160
   Asn Gly Val Asp Trp Asp Gln Arg Tyr Gln Glu Asn His Ile Phe Arg
                   165                 170                 175
   Phe Ala Asn Thr Asn Trp Asn Trp Arg Val Asp Glu Glu Asn Gly Asn
               180                 185                 190
   Tyr Asp Tyr Leu Leu Gly Ser Asn Ile Asp Phe Ser His Pro Glu Val
           195                 200                 205
   Gln Asp Glu Leu Lys Asp Trp Gly Ser Trp Phe Thr Asp Glu Leu Asp
       210                 215                 220
   Leu Asp Gly Tyr Arg Leu Asp Ala Ile Lys His Ile Pro Phe Trp Tyr
   225                 230                 235                 240
   Thr Ser Asp Trp Val Arg His Gln Arg Asn Glu Ala Asp Gln Asp Leu
                   245                 250                 255
   Phe Val Val Gly Glu Tyr Trp Lys Asp Asp Val Gly Ala Leu Glu Phe
               260                 265                 270
   Tyr Leu Asp Glu Met Asn Trp Glu Met Ser Leu Phe Asp Val Pro Leu
           275                 280                 285
   Asn Tyr Asn Phe Tyr Arg Ala Ser Gln Gln Gly Gly Ser Tyr Asp Met
       290                 295                 300
   Arg Asn Ile Leu Arg Gly Ser Leu Val Glu Ala His Pro Met His Ala
   305                 310                 315                 320
   Val Thr Phe Val Asp Asn His Asp Thr Gln Pro Gly Glu Ser Leu Glu
   325 330 335
   Ser Trp Val Ala Asp Trp Phe Lys Pro Leu Ala Tyr Ala Thr Ile Leu
   340 345 350
   Thr Arg Glu Gly Gly Tyr Pro Asn Val Phe Tyr Gly Asp Tyr Tyr Gly
   355 360 365
   Ile Pro Asn Asp Asn Ile Ser Ala Lys Lys Asp Met Ile Asp Glu Leu
   370 375 380
   Leu Asp Ala Arg Gln Asn Tyr Ala Tyr Gly Thr Gln His Asp Tyr Phe
   385 390 395 400
   Asp His Trp Asp Val Val Gly Trp Thr Arg Glu Gly Ser Ser Ser Arg
   405 410 415
   Pro Asn Ser Gly Leu Ala Thr Ile Met Ser Asn Gly Pro Gly Gly Ser
   420 425 430
   Lys Trp Met Tyr Val Gly Arg Gln Asn Ala Gly Gln Thr Trp Thr Asp
   435 440 445
   Leu Thr Gly Asn Asn Gly Ala Ser Val Thr Ile Asn Gly Asp Gly Trp
   450 455 460
   Gly Glu Phe Phe Thr Asn Gly Gly Ser Val Ser Val Tyr Val Asn Gln
   465 470 475 480
<210> 7
   <211> 22
   <212> DNA
   <213> Artificial Sequence
<220>
   <223> Oligonucleotide as PCR primer comA-AF
<400> 7
   aaggatgata atccgtcccg tg 22
<210> 8
   <211> 38
   <212> DNA
   <213> Artificial Sequence
<220>
   <223> Oligonucleotide as PCR primer comA-A/CmR
<400> 8
   gttatccgct cacaattcgg atggtcatca atcactag 38
<210> 9
   <211> 38
   <212> DNA
   <213> Artificial Sequence
<220>
   <223> Oligonucleotide as PCR primer comA-B/CmF
<400> 9
   cgtcgtgact gggaaaactg cgaaatcaga cggtgtac 38
<210> 10
   <211> 20
   <212> DNA
   <213> Artificial Sequence
<220>
   <223> Oligonucleotide as PCR primer comA-BR
<400> 10
   cgtcgcctat cggcgggcac 20
<210> 11
   <211> 25
   <212> DNA
   <213> Artificial Sequence
<220>
   <223> oligonucleotide as PCR primer yopO-AF
<400> 11
   atgtatatag gaggttggtg gtatg 25
<210> 12
   <211> 38
   <212> DNA
   <213> Artificial Sequence
<220>
   <223> oligonucleotide as PCR primer yopO-A/CMR
<400> 12
   gttatccgct cacaattcgc tctgacatgt caacctcc 38
<210> 13
   <211> 38
   <212> DNA
   <213> Artificial sequence
<220>
   <223> Oligonucleotide as PCR primer yopO-B/CmF
<400> 13
   cgtcgtgact gggaaaacag atgagaaagg aggagaag 38
<210> 14
   <211> 23
   <212> DNA
   <213> Artificial sequence
<220>
   <223> Oligonucleotide as PCR primer yopO-BR
<400> 14
   ataactgtta ctatataatg gcc 23
<210> 15
   <211> 20
   <212> DNA
   <213> Artificial sequence
<220>
   <223> Oligonucleotide as PCR primer treR-AF
<400> 15
   gctggggatg acgaatccga 20
<210> 16
   <211> 38
   <212> DNA
   <213> Artificial sequence
<220>
   <223> oligonucleotide as PCR primer treR-A/cmR
<400> 16
   gttatccgct cacaattctc accttcatta tggaccac 38
<210> 17
   <211> 38
   <212> DNA
   <213> Artificial Sequence
<220>
   <223> Oligonucleotide as PCR primer treR-B/CmF
<400> 17
   cgtcgtgact gggaaaacca ccgtctcgaca aattccg 38
<210> 18
   <211> 20
   <212> DNA
   <213> Artificial Sequence
<220>
   <223> Oligonucleotide as PCR primer treR-BR
<400> 18
   gttgccaagc gcgatatagg 20
<210> 19
   <211> 25
   <212> DNA
   <213> Artificial sequence
<220>
   <223> Oligonucleotide as PCR primer yvbA-AF
<400> 19
   tatacaggga ttatcagtat tgagc 25
<210> 20
   <211> 38
   <212> DNA
   <213> Artificial sequence
<220>
   <223> Oligonucleotide as PCR primer yvbA-A/CmR
<400> 20
   gttatccgct cacaattctt ttctccttgt tggatctg 38
<210> 21
   <211> 38
   <212> DNA
   <213> Artificial sequence
<220>
   <223> Oligonucleotide as PCR primer yvbA-B/CmF
<400> 21
   cgtcgtgact gggaaaacgg ggataacgat ttatgaag 38
<210> 22
   <211> 30
   <212> DNA
   <213> Artificial Sequence
<220>
   <223> Oligonucleotide as PCR primer yvbA-BR
<400> 22
   ttttgtaata atgatatgaa gctagtgttg 30
<210> 23
   <211> 20
   <212> DNA
   <213> Artificial Sequence
<220>
   <223> Oligonucleotide as PCR primer cspB-AF
<400> 23
   atatccagcc ctgcctcttc 20
<210> 24
   <211> 58
   <212> DNA
   <213> Artificial sequence
<220>
   <223> Oligonucleotide as PCR primer cspB-A/CmR
<400> 24
   ctgtgtgaaa ttgttatccg ctcacaattc gaaatttcct cctaaagcga tcataacg 58
<210> 25
   <211> 51
   <212> DNA
   <213> Artificial sequence
<220>
   <223> Oligonucleotide as PCR primer cspB-B/CmF
<400> 25
   gtcgttttac aacgtcgttg actgggaaaa cccacaagct gctaacgtta c 51
<210> 26
   <211> 20
   <212> DNA
   <213> Artificial sequence
<220>
   <223> oligonucleotide as PCR primer cspB-BR
<400> 26
   tcctgtttgg gctcctgttg 20
<210> 27
   <211> 26
   <212> DNA
   <213> Artificial Sequence
<220>
   <223> oligonucleotide as PCR primer yvaN-AF
<400> 27
   tgtttatgta tggcggcctg cgggac 26
<210> 28
   <211> 38
   <212> DNA
   <213> Artificial Sequence
<220>
   <223> Oligonucleotide as PCR primer yvaN-A/CmR
<400> 28
   gttatccgct cacaattcag ctttccatat atctcacc 38
<210> 29
   <211> 38
   <212> DNA
   <213> Artificial Sequence
<220>
   <223> oligonucleotide as PCR primer yvaN-B/cmF
<400> 29
   cgtcgtgact gggaaaacac ggtctgctga tgactgac 38
<210> 30
   <211> 20
   <212> DNA
   <213> Artificial sequence
<220>
   <223> oligonucleotide as PCR primer yvaN-BR
<400> 30
   gcgtttactt aagatgtcga 20
<210> 31
   <211> 29
   <212> DNA
   <213> Artificial Sequence
<220>
   <223> Oligonucleotide as PCR primer yttP-AF
<400> 31
   tttctagcgt ttcggcaaat tgagttaag 29
<210> 32
   <211> 38
   <212> DNA
   <213> Artificial sequence
<220>
   <223> oligonucleotide as PCR primer yttP-A/CmR
<400> 32
   gttatccgct cacaattcct tactttcata cggctcac 38
<210> 33
   <211> 38
   <212> DNA
   <213> Artificial Sequence
<220>
   <223> oligonucleotide as PCR primer yttP-B/CmF
<400> 33
   cgtcgtgact gggaaaacga gacgtggcgc tcaccaac 38
<210> 34
   <211> 29
   <212> DNA
   <213> Artificial Sequence
<220>
   <223> oligonucleotide as PCR primer yttP-BR
<400> 34
   cggattaaaa aaagaatatc gcggacagc 29
<210> 35
   <211> 20
   <212> DNA
   <213> Artificial Sequence
<220>
   <223> oligonucleotide as PCR primer yurK-AF
<400> 35
   tgccgctgcc cgccggagag 20
<210> 36
   <211> 38
   <212> DNA
   <213> Artificial Sequence
<220>
   <223> oligonucleotide as PCR primer yurK-A/CmR
<400> 36
   gttatccgct cacaattcaa ggtgtagaac ttccgttg 38
<210> 37
   <211> 38
   <212> DNA
   <213> Artificial Sequence
<220>
   <223> oligonucleotide as PCR primer yurK-B/CmF
<400> 37
   cgtcgtgact gggaaaacac catcaacagc ccctacac 38
<210> 38
   <211> 24
   <212> DNA
   <213> Artificial Sequence
<220>
   <223> Oligonucleotide as PCR primer yurK-BR
<400> 38
   tcaaataaag gcggcattca gtcc 24
<210> 39
   <211> 22
   <212> DNA
   <213> Artificial Sequence
<220>
   <223> oligonucleotide as PCR primer yoza-AF
<400> 39
   ataatggtat ccaaatccac gc 22
<210> 40
   <211> 38
   <212> DNA
   <213> Artificial Sequence
<220>
   <223> oligonucleotide as PCR primer yozA-A/CmR
<400> 40
   gttatccgct cacaattcat tcagtcatat gtatcacc 38
<210> 41
   <211> 38
   <212> DNA
   <213> Artificial Sequence
<220>
   <223> Oligonucleotide as PCR primer yozA-B/cmF
<400> 41
   cgtcgtgact gggaaaacga tccatcatac acagcatg 38
<210> 42
   <211> 28
   <212> DNA
   <213> Artificial Sequence
<220>
   <223> Oligonucleotide as PCR primer yozA-BR
<400> 42
   cacttctcaa cggaggggat ttcacatc 28
<210> 43
   <211> 20
   <212> DNA
   <213> Artificial sequence
<220>
   <223> oligonucleotide as PCR primer licR-AF
<400> 43
   taatggagga gagaaggccg 20
<210> 44
   <211> 38
   <212> DNA
   <213> Artificial sequence
<220>
   <223> Oligonucleotide as PCR primer licR-A/CmR
<400> 44
   gttatccgct cacaattcag tcgcccatga agcatgag 38
<210> 45
   <211> 42
   <212> DNA
   <213> Artificial sequence
<220>
   <223> oligonucleotide as PCR primer licR-B/CmF
<400> 45
   cgtcgtgact gggaaaacac caaaaaatgc tgagctgaca gc 42
<210> 46
   <211> 26
   <212> DNA
   <213> Artificial Sequence
<220>
   <223> Oligonucleotide as PCR primer licR-BR
<400> 46
   ttgccaatga tgaggaaaaa ggaacc 26
<210> 47
   <211> 26
   <212> DNA
   <213> Artificial Sequence
<220>
   <223> Oligonucleotide as PCR primer sigL-AF
<400> 47
   ctgaacgtcttgaataaaaaagcagg 26
<210> 48
   <211> 38
   <212> DNA
   <213> Artificial Sequence
<220>
   <223> oligonucleotide as PCR primer sigL-A/CmR
<400> 48
   gttatccgct cacaattcgc tgaagtttca tatccatc 38
<210> 49
   <211> 38
   <212> DNA
   <213> Artificial sequence
<220>
   <223> Oligonucleotide as PCR primer sigL-B/CmF
<400> 49
   cgtcgtgact gggaaaacat tccgtcatcg gcagcgag 38
<210> 50
   <211> 20
   <212> DNA
   <213> Artificial Sequence
<220>
   <223> oligonucleotide as PCR primer sigL-BR
<400> 50
   agcggtttac aagttggagg 20
<210> 51
   <211> 22
   <212> DNA
   <213> Artificial Sequence
<220>
   <223> Oligonucleotide as PCR primer mntR-AF
<400> 51
   atttcagaag gcatacttca ag 22
<210> 52
   <211> 38
   <212> DNA
   <213> Artificial Sequence
<220>
   <223> oligonucleotide as PCR primer mntR-A/CmR
<400> 52
   gttatccgct cacaattcca tacttggtgt tgtcatcg 38
<210> 53
   <211> 40
   <212> DNA
   <213> Artificial sequence
<220>
   <223> oligonucleotide as PCR primer mntR-B/CmF
<400> 53
   cgtcgtgact gggaaaacca taatcagtaa aaaggcggtc 40
<210> 54
   <211> 20
   <212> DNA
   <213> Artificial sequence
<220>
   <223> oligonucleotide as PCR primer mntR-BR
<400> 54
   ttctgaccgc tctggcaacc 20
<210> 55
   <211> 20
   <212> DNA
   <213> Artificial sequence
<220>
   <223> Oligonucleotide as PCR primer glcT-AF
<400> 55
   ataatgcccg cttcccaacc 20
<210> 56
   <211> 38
   <212> DNA
   <213> Artificial sequence
<220>
   <223> oligonucleotide as PCR primer glcT-A/cmR
<400> 56
   gttatccgct cacaattccg atcctcagct cctttgtc 38
<210> 57
   <211> 38
   <212> DNA
   <213> Artificial Sequence
<220>
   <223> oligonucleotide as PCR primer glcT-B/CmF
<400> 57
   cgtcgtgact gggaaaactc atctgatacc gattaacc 38
<210> 58
   <211> 20
   <212> DNA
   <213> Artificial Sequence
<220>
   <223> Oligonucleotide as PCR primer glcT-BR
<400> 58
   caactgaatc cgaaggaatg 20
<210> 59
   <211> 20
   <212> DNA
   <213> Artificial Sequence
<220>
   <223> oligonucleotide as PCR primer yvdE-AF
<400> 59
   tcggggtcat gccgagcggt 20
<210> 60
   <211> 38
   <212> DNA
   <213> Artificial Sequence
<220>
   <223> Oligonucleotide as PCR primer yvdE-A/CmR
<400> 60
   gttatccgct cacaattcca atgttgccat tttcatcc 38
<210> 61
   <211> 38
   <212> DNA
   <213> Artificial sequence
<220>
   <223> Oligonucleotide as PCR primer yvdE-B/CmF
<400> 61
   cgtcgtgact gggaaaactt gtacgagaat caacgctg 38
<210> 62
   <211> 20
   <212> DNA
   <213> Artificial Sequence
<220>
   <223> Oligonucleotide as PCR primer yvdE-BR
<400> 62
   cacggcaatg cattcttcgg 20
<210> 63
   <211> 21
   <212> DNA
   <213> Artificial sequence
<220>
   <223> Oligonucleotide as PCR primer ykvE-AF
<400> 63
   agatctgtcg gccaggttta c 20
<210> 64
   <211> 38
   <212> DNA
   <213> Artificial sequence
<220>
   <223> Oligonucleotide as PCR primer ykvE-A/CmR
<400> 64
   gttatccgct cacaattctg atttttctgt catgtctc 61
<210> 65
   <211> 38
   <212> DNA
   <213> Artificial sequence
<220>
   <223> oligonucleotide as PCR primer ykvE-B/CmF
<400> 65
   cgtcgtgact gggaaaacgg tagagatgtg caccgaaa 38
<210> 66
   <211> 20
   <212> DNA
   <213> Artificial sequence
<220>
   <223> oligonucleotide as PCR primer ykvE-BR
<400> 66
   gagtcagacg gcatcgatga 20
<210> 67
   <211> 23
   <212> DNA
   <213> Artificial Sequence
<220>
   <223> Oligonucleotide as PCR primer slr-AF
<400> 67
   ttctgattca ttttcactgc tgg 23
<210> 68
   <211> 38
   <212> DNA
   <213> Artificial sequence
<220>
   <223> Oligonucleotide as PCR primer slr-A/CmR
<400> 68
   gttatccgct cacaattcaa cggataattc ttccaatc 38
<210> 69
   <211> 37
   <212> DNA
   <213> Artificial sequence
<220>
   <223> Oligonucleotide as PCR primer slr-B/CmF
<400> 69
   cgtcgtgact gggaaaactg tccatgaagt caaatcc 37
<210> 70
   <211> 20
   <212> DNA
   <213> Artificial Sequence
<220>
   <223> Oligonucleotide as PCR primer slr-BR
<400> 70
   cgctgaaata ttctctcgca 20
<210> 71
   <211> 21
   <212> DNA
   <213> Artificial sequence
<220>
   <223> Oligonucleotide as PCR primer rocR-AF
<400> 71
   cgccgctttc accgcggatt c 21
<210> 72
   <211> 38
   <212> DNA
   <213> Artificial Sequence
<220>
   <223> Oligonucleotide as PCR primer rocR-A/CmR
<400> 72
   gttatccgct cacaattcct ttgaccactg tatgaacc 38
<210> 73
   <211> 38
   <212> DNA
   <213> Artificial Sequence
<220>
   <223> oligonucleotide as PCR primer rocR-B/CmF
<400> 73
   cgtcgtgact gggaaaacac tcgtctaacg aataatcc 38
<210> 74
   <211> 20
   <212> DNA
   <213> Artificial sequence
<220>
   <223> Oligonucleotide as PCR primer rocR-BR
<400> 74
   tgtcatcacg gaatttgacg 20
<210> 75
   <211> 30
   <212> DNA
   <213> Artificial sequence
<220>
   <223> Oligonucleotide as PCR primer ccpA-AF
<400> 75
   ccaaattatc ctttgtgagc gcggaatcag 30
<210> 76
   <211> 38
   <212> DNA
   <213> Artificial Sequence
<220>
   <223> Oligonucleotide as PCR primer ccpA-A/CmR
<400> 76
   gttatccgct cacaattccg tagatcgtaa tattgctc 38
<210> 77
   <211> 38
   <212> DNA
   <213> Artificial Sequence
<220>
   <223> oligonucleotide as PCR primer ccpA-B/CmF
<400> 77
   cgtcgtgact gggaaaacag cttagaaagt caaccaag 38
<210> 78
   <211> 20
   <212> DNA
   <213> Artificial sequence
<220>
   <223> Oligonucleotide as PCR primer ccpA-BR
<400> 78
   tttgagcatc agcacaagcc 20
<210> 79
   <211> 21
   <212> DNA
   <213> Artificial sequence
<220>
   <223> Oligonucleotide as PCR primer yaaT-AF
<400> 79
   tgtagcagaa gcagtcgaat t 21
<210> 80
   <211> 40
   <212> DNA
   <213> Artificial sequence
<220>
   <223> oligonucleotide as PCR primer yaaT-A/Cm2R
<400> 80
   ctaatgggtg ctttagttga caattacgca gctgtcatgt 40
<210> 81
   <211> 41
   <212> DNA
   <213> Artificial sequence
<220>
   <223> Oligonucleotide as PCR primer yaaT-B/Cm2F
<400> 81
   ctgccccgtt agttgaagaa ctgataaacc gtgaaaaagt g 41
<210> 82
   <211> 20
   <212> DNA
   <213> Artificial sequence
<220>
   <223> oligonucleotide as PCR primer yaaT-RV
<400> 82
   cctttgaaaa aggctcccgt 20
<210> 83
   <211> 29
   <212> DNA
   <213> Artificial Sequence
<220>
   <223> oligonucleotide as PCR primer yyaA-AF
<400> 83
   gttttccaag tctgccgata aaaatatgc 29
<210> 84
   <211> 38
   <212> DNA
   <213> Artificial sequence
<220>
   <223> Oligonucleotide as PCR primer yyaA-A/CmR
<400> 84
   gttatccgct cacaattcat gcttcatgta cctacacc 38
<210> 85
   <211> 38
   <212> DNA
   <213> Artificial Sequence
<220>
   <223> oligonucleotide as PCR primer yyaA-B/CmF
<400> 85
   cgtcgtgact gggaaaacca attaacgatt cgcatacc 38
<210> 86
   <211> 30
   <212> DNA
   <213> Artificial sequence
<220>
   <223> Oligonucleotide as PCR primer yyaA-BR
<400> 86
   aaaaagaaga agtcacagta cagaacgtgg 30
<210> 87
   <211> 23
   <212> DNA
   <213> Artificial Sequence
<220>
   <223> oligonucleotide as PCR primer yycH-AF
<400> 87
   atttttcgcc atcttgaatt ttc 23
<210> 88
   <211> 40
   <212> DNA
   <213> Artificial Sequence
<220>
   <223> Oligonucleotide as PCR primer yycH-A/Cm2R
<400> 88
   ctaatgggtg ctttagttgg atgatcctct cgttgaactg 40
<210> 89
   <211> 39
   <212> DNA
   <213> Artificial Sequence
<220>
   <223> Oligonucleotide as PCR primer yycH-B/Cm2F
<400> 89
   ctgccccgtt agttgaaggg atgagccttc agaaaagtt 39
<210> 90
   <211> 20
   <212> DNA
   <213> Artificial sequence
<220>
   <223> Oligonucleotide as PCR primer yycH-BR
<400> 90
   gccggacaga gatctgtatg 20
<210> 91
   <211> 45
   <212> DNA
   <213> Artificial sequence
<220>
   <223> Oligonucleotide as PCR primer yacP-B/Cm4F
<400> 91
   gaagaaggtt tttatgttga cgcttttttg cccaatactg tataa 45
<210> 92
   <211> 45
   <212> DNA
   <213> Artificial sequence
<220>
   <223> Oligonucleotide as PCR primer yacP-B/Cm4R
<400> 92
   caaaaaagcg tcaacataaa aaccttcttc aactaacggg gcagg 45
<210> 93
   <211> 30
   <212> DNA
   <213> Artificial sequence
<220>
   <223> Oligonucleotide as PCR primer yacP-BR
<400> 93
   aagacgagta cttttctctc taaatcactt 30
<210> 94
   <211> 30
   <212> DNA
   <213> Artificial sequence
<220>
   <223> oligonucleotide as PCR primer yacP-AF
<400> 94
   aactcgatca aatggtgaca ggacagcatc 30
<210> 95
   <211> 45
   <212> DNA
   <213> Artificial sequence
<220>
   <223> oligonucleotide as PCR primer yacP-A/Cm4F
<400> 95
   ggagaataaa gaccctcttc aactaaagca cccattagtt caaca 45
<210> 96
   <213> 45
   <212> DNA
   <213> Artificial Sequence
<220>
   <223> oligonucleotide as PCR primer yacP-A/Cm4R
<400> 96
   tgctttagtt gaagagggtc tttattctcc cacagggttt cgttt 45
<210> 97
   <211> 45
   <212> DNA
   <213> Artificial Sequence
<220>
   <223> oligonucleotide as PCR primer hprK-B/Cm4F
<400> 97
   tttttatatt acagcgagtt ggcgttaaat gaatgaagcg ataga 45
<210> 98
   <211> 45
   <212> DNA
   <213> Artificial Sequence
<220>
   <223> Oligonucleotide as PCR primer hprK-B/Cm4R
<400> 98
   atttaacgcc aactcgctgt aatataaaaa ccttcttcaa ctaac 45
<210> 99
   <211> 30
   <212> DNA
   <213> Artificial Sequence
<220>
   <223> oligonucleotide as PCR primer hprK-BR
<400> 99
   ttgattgatg ataaattcag gcaggtgcag 30
<210> 100
   <211> 30
   <212> DNA
   <213> Artificial sequence
<220>
   <223> Oligonucleotide as PCR primer hprK-AF
<400> 100
   caaagcttga gaaatgttcc catgctcttg 30
<210> 101
   <211> 45
   <212> DNA
   <213> Artificial Sequence
<220>
   <223> Oligonucleotide as PCR primer hprK-A/Cm4F
<400> 101
   caggaggaac atatctcttc aactaaagca cccattagtt caaca 45
<210> 102
   <211> 45
   <212> DNA
   <213> Artificial sequence
<220>
   <223> oligonucleotide as PCR primer hprK-A/Cm4R
<400> 102
   tgctttagtt gaagagatat gttcctcctg ttccgggctg ccccg 45
<210> 103
   <211> 25
   <212> DNA
   <213> Artificial Sequence
<220>
   <223> Oligonucleotide as PCR primer rsiX-AF
<400> 103
   attccagtta ctcgtaatat agttg 25
<210> 104
   <211> 38
   <212> DNA
   <213> Artificial Sequence
<220>
   <223> Oligonucleotide as PCR primer rsiX-A/CmR
<400> 104
   gttatccgct cacaattcac ttcatcatcc attagctc 38
<210> 105
   <211> 38
   <212> DNA
   <213> Artificial Sequence
<220>
   <223> oligonucleotide as PCR primer rsiX-B/CmF
<400> 105
   cgtcgtgact gggaaaacct gctccaaatc cgatttcc 38
<210> 106
   <211> 23
   <212> DNA
   <213> Artificial Sequence
<220>
   <223> oligonucleotide as PCR primer rsix-BR
<400> 106
   gtcctgcatt tttcgaagtc tgg 23
<210> 107
   <211> 30
   <212> DNA
   <213> Artificial Sequence
<220>
   <223> oligonucleotide as PCR primer yhdK-AF
<400> 107
   tacacatcct tcaaacaagt ctgaacaaac 30
<210> 108
   <211> 45
   <212> DNA
   <213> Artificial Sequence
<220>
   <223> oligonucleotide as PCR primer yhdK-A/cm4R
<400> 108
   tgctttagtt gaagattacc agttccataa ttccacctcg ccgac 45
<210> 109
   <211> 45
   <212> DNA
   <213> Artificial Sequence
<220>
   <223> oligonucleotide as PCR primer yhdK-B/Cm4F
<400> 109
   tttttatatt acagcgtgtg tataccattg tatctgtaga tacga 45
<210> 110
   <211> 30
   <212> DNA
   <213> Artificial Sequence
<220>
   <223> oligonucleotide as PCR primer yhdK-BR
<400> 110
   gctatgatca ttgtaacgaa aggaaagggg 30
<210> 111
   <211> 45
   <212> DNA
   <213> Artificial Sequence
<220>
   <223> Oligonucleotide as PCR primer yhdK-A/cm4F
<400> 111
   ttatggaact ggtaatcttc aactaaagca cccattagtt caaca 45
<210> 112
   <211> 45
   <212> DNA
   <213> Artificial sequence
<220>
   <223> oligonucleotide as PUTT primer yhdK-B/Cm4R
<400> 112
   caatggtata cacacgctgt aatataaaaa ccttcttcaa ctaac 45
<210> 113
   <211> 30
   <212> DNA
   <213> Artificial sequence
<220>
   <223> oligonucleotide as PCR primer ylbO-AF
<400> 113
   aatctgaaca agaaaaagga gctgctcctc 30
<210> 114
   <211> 45
   <212> DNA
   <213> Artificial Sequence
<220>
   <223> Oligonucleotide as PCR primer ylbO-A/Cm4R
<400> 114
   tgctttagtt gaagaattca atctccctcc atgtcagctt attta 45
<210> 115
   <211> 45
   <212> DNA
   <213> Artificial Sequence
<220>
   <223> Oligonucleotide as PCR primer ylbO-B/Cm4F
<400> 115
   tttttatatt acagcagaaa cgcctgaaat gaaccggccc tatag 45
<210> 116
   <211> 30
   <212> DNA
   <213> Artificial Sequence
<220>
   <223> Oligonucleotide as PCR primer ylbO-BR
<400> 116
   tgtttgacaa aggtagaacg tctgcttatc 30
<210> 117
   <211> 45
   <212> DNA
   <213> Artificial Sequence
<220>
   <223> Oligonucleotide as PCR primer ylbO-A/Cm4F
<400> 117
   ggagggagat tgaattcttc aactaaagca cccattagtt caaca 45
<210> 118
   <211> 45
   <212> DNA
   <213> Artificial Sequence
<220>
   <223> Oligonucleotide as PCR primer ylbO-B/Cm4R
<400> 118
   atttcaggcg tttctgctgt aatataaaaa ccttcttcaa ctaac 45
<210> 119
   <211> 18
   <212> DNA
   <213> Artificial Sequence
<220>
   <223> oligonucleotide as PCR primer CmF
<400> 119
   gaattgtgag cggataac 18
<210> 120
   <211> 18
   <212> DNA
   <213> Artificial sequence
<220>
   <223> oligonucleotide as PCR primer cmR
<400> 120
   gttttcccag tcacgacg 18
<210> 121
   <211> 19
   <212> DNA
   <213> Artificial sequence
<220>
   <223> oligonucleotide as PCR primer Cm2F
<400> 121
   caactaaagc acccattag 19
<210> 122
   <211> 18
   <212> DNA
   <213> Artificial sequence
<220>
   <223> oligonucleotide as PCR primer cm2R
<400> 122
   cttcaactaa cggggcag 18

## Description

### Technical Field

The present invention relates to a recombinant microorganism which may be used to produce useful proteins or polypeptides, as well as to such proteins and polypeptides.

### Technical Background

Microorganisms are widely used for industrially producing a broad range of useful substances, including alcoholic beverages, certain types of foods such as *miso* and *shoyu*, amino acids, organic acids, nucleic-acid-related substances, antibiotics, sugars, lipids, and proteins. These substances also find diversified uses, including foods, pharmaceuticals, detergents, products for daily use such as cosmetics, and a variety of chemical raw materials.

In industrial production of useful substances by use of microorganisms, improvement of productivity is one major topic of interest, and one approach therefor is breeding of microorganisms through mutagenesis or other genetic means. Recently, in particular, with advancement of microbial genetics and biotechnology, more efficient breeding of useful microorganisms is performed through gene recombination techniques, and in association therewith, host microorganisms for obtaining recombinant genes are under development. For example, *Bacillus subtilis* Marburg No. 168, which has already been confirmed to be safe and have excellent characteristics as a host microorganism, has been further improved.

However, microorganisms inherently possess diversified genes so that they can cope with environmental changes in the natural world, and thus, they do not necessarily exhibit high production efficiency of proteins or similar substances in industrial production, where only limited production media are employed.

### Disclosure of the Invention

The present invention is characterized by the embodiments as defined in the claims. Thus, it relates to the following items:
1. A recombinant microorganism prepared by transferring, to a mutant strain of a microorganism from which any of *Bacillus subtilis* genes cspB, sigL, glcT, rocR and ccpA have been deleted or knocked out, a gene encoding a heterologous protein or polypeptide,
   wherein the microorganism is *Bacillus subtilis*, wherein three regions constituted by a transcription initiation regulatory region, a translation initiation regulatory region, and a secretion signal region are ligated to an upstream region of the gene encoding a heterologous protein or polypeptide, wherein the transcription initiation regulatory region and the translation initiation regulatory region are each derived from a 0.6 to 1 kb region upstream of the cellulase gene of a bacterium belonging to the genus Bacillus, and wherein the three regions constituted by the transcription initiation regulatory region, the translation initiation regulatory region, and the secretion signal region are
   (a) a nucleotide sequence of base numbers 1 to 659 of a cellulase gene of SEQ ID NO: 1;
   (b) a nucleotide sequence of base numbers 1 to 696 of a cellulase gene of SEQ ID NO: 3; or
   (c) a DNA fragment having a nucleotide sequence having 80% homology with either of the nucleotide sequences of (a) or (b).
2. A method for producing a protein or polypeptide by use of a recombinant microorganism as defined in item 1.

The present disclosure provides a recombinant microorganism prepared by transferring, to a mutant strain of microorganism from which any of *Bacillus subtilis* genes *comA, yopO, treR, yvbA, cspB, yvaN, yttP, yurK, yozA, licR, sigL, mntR, glcT, yvdE, ykvE, slr, rocR, ccpA, yaaT, yyaA, yycH; yacP, hprK, rsiX, yhdK,* and *ylbO,* or one or more genes functionally equivalent to any of these genes have been deleted or knocked out, a gene encoding a heterologous protein or polypeptide.

### Brief Description of the Drawings

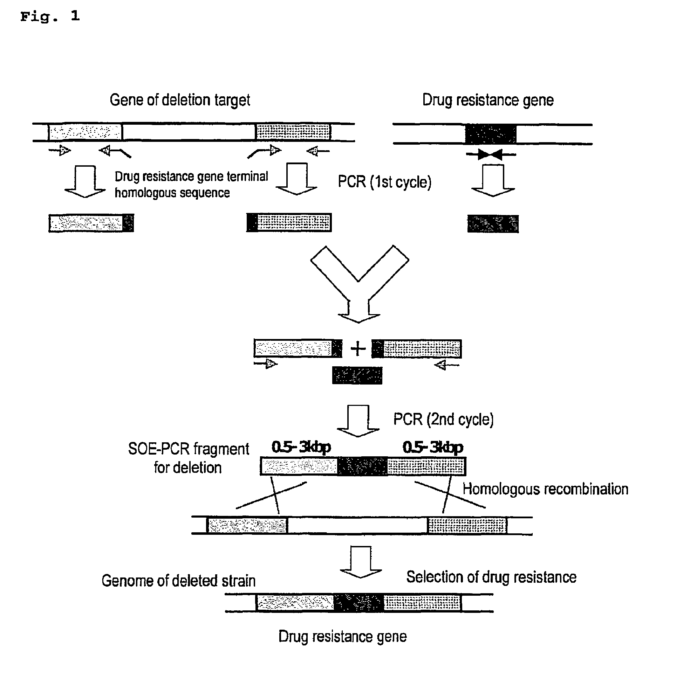

Fig. 1 schematically shows a method for preparing a DNA fragment for deleting a gene through SOE-PCR (SOE: splicing by overlap extension) (see Gene, 77, 61 (1989), and a method for deleting a target gene (replacing the target gene with a drug resistance gene) through use of the DNA.

### Modes for Carrying out the Invention

The present invention is directed to a recombinant microorganism obtained by transferring, into a host microorganism capable of producing protein or polypeptide with increased productivity, a gene encoding a protein or polypeptide, and to a method for producing a protein or polypeptide by use of the recombinant microorganism.

The present inventors have conducted extensive studies on, among many different genes encoded on the genome of a microorganism, genes which are not needed in or which are detrimental to the production of useful proteins or polypeptides, and have found that, when a gene encoding a target protein or polypeptide is transferred to the microorganism *Bacillus subtilis* after a specific gene is deleted or knocked out from the genome of the microorganism, productivity of the target protein or polypeptide is enhanced as compared with the case before the deletion or knocking out.

In the microorganism of the present invention, since genes which are unnecessary in or detrimental to the production or a target protein or polypeptide are deleted or knocked out, waste of culture media, including energy loss, production of byproducts, and reduced specific production rate, is significantly reduced, and in addition, protein and polypeptide can be produced over a prolonged period, whereby a target product can be produced with high efficiency.

In the present invention, homology between amino acid sequences and that between nucleic acid sequences are both determined by use of the Lipman-Pearson method (Science, 227, 1935 (1985)). Specifically, calculation is performed by use of a homology analysis program (Search Homology) developed by genetic information processing software, Genetyx-Win (Software Development Co., Ltd.), with ktup (the unit size to be compared) being set 2.

The parent microorganism for constructing the microorganism of the present invention is *Bacillus subtilis* having genes or genes functionally equivalent thereto as shown in Table 1, wherein the gene may be of wild-type or a mutant.

*Bacillus subtilis* is used, from the viewpoint that complete genomic information of this microorganism has already been obtained, and thus genetic engineering techniques and genomic engineering techniques have been established, and that the microorganism has ability to secrete the produced protein extracellularly.

Examples of the target protein or polypeptide to be produced by use of the microorganism of the present invention include enzymes, physiologically active substances, and other proteins and polypeptides which find utility in foods, pharmaceuticals, cosmetics, detergents, fiber-treating agents, clinical assay agents, etc.

Taking *Bacillus subtilis,* which is known to have 4,106 genes on the genome, as an example, one or more genes which are to be deleted or knocked out are any of the *Bacillus subtilis* genes shown in Table 1, or are selected from among the genes functionally equivalent thereto. The present inventors have found that such genes do not directly participate in production of the target protein or polypeptide and are unnecessary for the growth of microorganism in ordinary industrial production media.

The names, numbers, and functions of respective genes in the Tables contained herein conform with the *Bacillus subtilis* genome data reported in Nature, 390, 249-256 (1997) and made public by JAFAN (Japan Functional Analysis Network for *Bacillus subtilis;* BSORF DB) on the Internet (http://bacillus.genome.ad.jp/, renewed June 17, 2003).

**Table 1**

| Name of the gene | Gene ID | Functions or other information of the gene |
|---|---|---|
| *ComA* | BG10381 | two-component response regulator |
| *yopO* | BG13648 | deduced transcriptional regulator, spβ prophage protein |
| *treR* | BG11011 | trehalose operon transcriptional repressor (GntR family) |
| *yvbA* | BG14078 | deduced transcriptional regulator (ArsR family) |
| *cspB* | BG10824 | cold shock-related major factor |
| *yvaN* | BG14069 | deduced transcriptional regulator |
| *yttP* | BG13927 | deduced transcriptional regulator (TetR family) |
| *yurK* | BG13997 | deduced transcriptional regulator (GntR family) |
| *yozA* | BG13748 | deduced transcriptional regulator (ArsR family) |
| *licR* | BG11346 | transcriptional regulator (antiterminator), lichenan operon (*licBCAH*) regulation |
| *sigL* | BG10748 | RNA polymerase σ factor (o54) |
| *mntR* | BG11702 | manganese transport regulator |
| *glcT* | BG12593 | transcriptional regulator essential to expression of *ptsGHI* operon (BglG family, antiterminator) |
| *yvdE* | BG12414 | deduced transcriptional regulator (LacI family) |
| *ykvE* | BG13310 | deduced transcriptional regulator (MarR family) |
| *slr* | BG11858 | transcriptional activator for competence- or sporulation-related genes |
| *rocR* | BG10723 | transcriptional activator for arginine-assimilating operon (NtrC family) |
| *ccpA* | BG10376 | carbon source catabolism reppression-related transcriptional regulator (Lacl family) |
| *yaaT* | BG10096 | type-II signal peptidase-like protein |
| *yyaA* | BG10057 | DNA-binding protein SpoOJ-like protein |
| *yycH* | BG11462 | Function unknown (homologous gene has been found in other organisms) |
| *yacP* | BG10158 | Function unknown (homologous gene has been found in other organisms) |
| *hprK* | BG14125 | Hpr protein Ser residue phosphoenzyme/dephosphoenzyme |
| *rsiX* | BG10537 | anti σX factor |
| *yhdK* | BG13017 | Function unknown, related to repression of σM factor expression |
| *ylbO* | BG13367 | expression regulator for gene in σE-related metrocytein |

Genes derived from other microorganisms, preferably from bacteria belonging to the genus *Bacillus,* which have the same functions as any of the *Bacillus subtilis* genes shown in Table 1, or have 70% or more homology with the nucleotide sequence of any of the genes shown in Table 1, preferably 80% or more homology, more preferably 90% or more, further preferably 95% or more, yet more preferably 98% or more, should be interpreted to be functionally equivalent to the genes shown in Table 1, and thus to constitute the genes which are to be deleted or knocked out according to the present invention. In this connection, homology of nucleotides is computed by use of the Lipman-Pearson method (Science, 227, 1435, 1985).

Many of the genes shown in Table 1 which encode *Bacillus subtilis* are regulatory genes participating in activation or suppression of expression of a variety of genes, or genes deduced to be such regulatory genes. The present invention has been attained on the basis of this finding; i.e., the presence of regulatory genes unnecessary in or detrimental to production of protein or polypeptide has now been unveiled in the present invention.

Notably, attention is drawn to the fact that many of the listed "unnecessary" or "detrimental" genes are regulatory genes participating in sugar intake or metabolism, as exemplified by the *glcT* gene, which acts as an anti-terminator for a glucose PTS intake operon; the *licT* gene, which acts as an anti-terminator for a lichenan hydrolysis operon; the *treR* gene, which acts as a repressor of trehalose intake and metabolism; and the *hprK* gene and *ccpA* gene, which relate to glucose catabolite repression.

Also, in addition to the regulatory genes involved in sugar intake and metabolism, the *rocR* gene participating in activation of arginine assimilation, and competence-related *comA* gene and *slr* gene, which are also regulatory genes, may be deleted or knocked out, to thereby improve productivity of protein or polypeptide.

The genes shown in Table 1 include the *yhdK* gene, and the *rsiX* gene encoding the anti-EC F sigma factor which suppresses expression of an ECF sigma factor, sigma x. The *yhdK* gene has been reported to participate in suppression of sigma M (Mol. Microbiol., 32, 41, 1999). The *sigL* gene, which encodes sigma L, is also included in the genes of Table 1. This suggests that expression of a gene under regulation by sigma X or sigma M is favorable for production of protein, and conversely, some gene expression under regulation by sigma L is unfavorable.

By deleting or knocking out one or more genes selected from the above-mentioned genes, expression which is unnecessary in or harmful to the production of protein or polypeptide can be prevented, leading to enhanced productivity in such production of protein or polypeptide.

The number of gene(s) to be deleted or knocked out is one or more, preferably two or more, more preferably three or more, even more preferably 5 or more. When a microorganism of the present invention is constructed, deletion or inactivation of a gene or genes other than those mentioned above is possible. In such a case, a more improved effect is expected. An alternative method for achieving the present invention is inactivation, or knocking out, of a target gene by inserting thereto a DNA fragment of another origin or introducing a mutation to the transcription/translation-initiation region of the gene. Preferably, however, the target genes are physically deleted.

In an example procedure for deleting or knocking out the genes, any of the target genes shown in Table 1 is deleted or knocked out according to a plan which has been set up in advance. Alternatively, randomized deletion of genes or mutation by way of knocking out is performed, followed by evaluation on protein productivity and gene analysis.

The target gene may be deleted or knocked out through homologous recombination. That is, a DNA fragment containing a portion of the target gene is cloned with an appropriate plasmid vector to thereby obtain a ring-shaped recombinant plasmid, and the resultant plasmid is transferred into cells of a parent microorganism. Thereafter, through homologous recombination effected in a partial region of the target gene, the target gene on the genome of the parent microorganism is cleaved, thereby completing inactivation of the target gene. Alternatively, the target gene is knocked out by substitution or insertion of a base, or a linear DNA fragment containing a region outside the target gene sequence but not containing the target gene may be constituted through PCR or a similar method, and the thus-engineered gene or fragment is transferred into a cell of a parent microorganism. At two sites outside the mutation within the target gene in the genome of the parent microorganism genome, or at two regions outside the target gene sequence, double crossing-over homologous recombination is caused to occur, to thereby attain substitution with a gene fragment in which the target gene on the genome is deleted or knocked out.

Particularly when the parent microorganism used to construct the microorganism of the present invention is *Bacillus subtilis,* since several reports have already described methods for deleting or knocking out the target gene (see, for example, Mol. Gen. Genet., 223, 268 1990), repetition of any of such methods may be followed, to thereby produce a host microorganism of the present invention.

Randomized gene deletion or inactivation may be performed through use of a method similar to the above-described method for inducing homologous recombination by use of a randomly cloned DNA fragment, or by way of irradiation of a parent microorganism with gamma rays or similar rays.

Next will be described in more detail a deletion method employing double crossing over by use of a DNA fragment designed for the deletion purpose, the DNA fragment being prepared through SOE-PCR (Gene, 77, 61, 1989). However, in the present invention, the method for deleting genes is not limited to only the below-described method.

The DNA fragment use for the deletion purpose is a fragment constructed such that a drug resistant marker gene is inserted between a ca. 0.5 to 3 kb upstream sequence which flanks and is upstream of the gene to be deleted, and a ca. 0.5 to 3 kb downstream sequence which flanks and is downstream of the same gene. In the first cycle of PCR, the following three fragments are prepared: the upstream and the downstream fragments, which are to be deleted, and the drug resistant marker gene. The primers to be used in this step may, for example, be those specifically designed so that an upstream 10-30 base pair sequence of a drug resistance gene is added to the lower end of the upstream fragment, and a downstream 10-30 base pair sequence of the drug resistance marker gene is added to the upper end of the downstream fragment (Fig. 1).

Next, using three PCR fragments prepared in the first cycle as templates, the second cycle of PCR is performed by use of an upper primer of the upstream fragment and a lower primer of the downstream fragment. This step causes annealing with the drug resistance marker gene fragment in the sequence of the above-engineered drug resistance marker gene, and through PCR amplification, there can be obtained a DNA fragment with the drug resistance marker gene inserted between the upstream fragment and the downstream fragment (Fig. 1).

When a chloramphenicol-resistant gene is employed as a drug resistance marker gene, a DNA fragment for deleting a gene can be obtained through SOE-PCR under typical conditions described in literature (see, for example, PCR Protocols. Current Methods and Applications, Edited by B. A. White, Humana Press, pp. 251 (1993), Gene, 77, 61, 1989), by use of a primer set such as that shown in Table 2 and a conventional enzyme kit for PCR (e.g., Pyrobest DNA Polymerase (product of Takara Shuzo)).

When the thus-obtained DNA fragment for effecting gene deletion is introduced into cells through the competent method or a similar method, intracellular genetic recombination occurs in homologous regions which are present upstream and downstream of the gene to be deleted. Thus, cells in which the target gene has been substituted by a drug resistance gene can be selectively separated through employment of a drug resistance marker (Fig. 1). Specifically, when a DNA fragment for gene deletion prepared by use of a primer set shown in Table 2 is introduced into cells, colonies which have grown on an agar culture medium containing chloramphenicol are separated, and deletion of the target gene by way of substitution by the chloramphenicol-resistant gene is confirmed through an appropriate method such as PCR employing a genome as a template.

Subsequently, when a gene encoding a target protein or polypeptide is transferred to a host mutant microorganism strain from which any of the *Bacillus subtilis* genes shown in Table 1, or one or more genes selected from among the genes corresponding thereto has been deleted or knocked out, the microorganism of the present invention can be obtained.

No particular limitation is imposed on the gene encoding the target protein or polypeptide. Examples of the protein and polypeptide include physiologically-active peptides and enzymes for industrial purposes such as detergents, foods, fibers, feeds, chemicals, medicine, and diagnostic agents. Industrial enzymes may be functionally grouped into oxidoreductases, transferases, hydrolases, lyases, isomerases, and ligases/synthetases. Preferably, hydrolases such as cellulase, α-amylase, and protease may be used. Specific examples include cellulase belonging to family 5 in the classification of hydrolase (Bioche M. J., 280, 309, 1991); in particular, cellulase derived from a microorganism, more particularly cellulase derived from the genus *Bacillus.* Other specific examples of the types of industrial enzymes include alkaline cellulase which is derived from the genus *Bacillus* and has an amino-acid of SEQ ID NOs: 2 or 4, and cellulase which has an another amino-acid sequence having 70% homology with said amino-acid sequence, preferably 80% homology, more preferably 90%, further preferably 95%, still further preferably 98% or more.

Specific examples of α-amylase include α-amylase derived from a microorganism, preferably liquefied amylase derived from the genus *Bacillus.* More specific examples include alkaline amylase which is derived from the genus *Bacillus* and has an amino-acid sequence of SEQ ID NO: 6, and amylase which has another amino-acid sequence having 70% homology with said amino-acid sequence, preferably 80% homology, more preferably 90%, further preferably 95%, particularly preferably 98% or more. The homology of the amino-acid sequence is calculated by the Lipman-Pearson method (Science, 227, 1435 (1985)). Specific examples of protease include serine protease and metallo-protease which are derived from microorganisms, particularly those belonging to the genus *Bacillus.*

Preferably, a gene coding for a target protein or polypeptide has, on its upstream region thereof, one or more regulatory regions relating to transcription, translation, or secretion of the gene (specially, one or more regions selected from among a transcription initiation regulatory region including a promoter and a transcription initiation site; a translation initiation region including a ribosome-binding site and a start codon; and a secretion signal peptide region) properly ligated thereto. Preferably, it is preferred that three regions consisting of the transcription initiation regulatory region, the translation initiation regulatory region, and the secretion signal region be ligated to the target gene. Further preferably, the secretion signal peptide region is one that originates from the cellulase gene of a microorganism belonging to the genus *Bacillus,* and the transcription initiation region and the translation initiation region is a 0.6 to 1 kb region upstream of the cellulase gene. In one preferred example, a transcription initiation regulatory region, a translation initiation region, and a secretion signal peptide region of a cellulase gene derived from a microorganism belonging to the genus *Bacillus* disclosed in, for example, Japanese Patent Application Laid-Open (*kokai*) Nos. 2000-210081 and 190793/1990; i.e., a cellulase gene derived from KSM-S237 strain (FERM BP-7875) or KSM-64 strain (FERM BP-2886), is properly ligated to a structural gene of the target protein or polypeptide. More specifically, preferred DNA fragments to be ligated include a nucleotide sequence of base numbers 1 to 659 of SEQ ID NO: 1; a nucleotide sequence of base numbers 1 to 696 of a cellulase gene of SEQ ID NO: 3; a DNA fragment having a nucleotide sequence having 70% homology with any one of said nucleotide sequences, preferably 80% homology, more preferably 90%, further preferably 95%, even more preferably 98% or more; or a DNA fragment having a nucleotide sequence lacking a portion of any one of said nucleotide sequences. Preferably, one of these DNA fragments is properly ligated to a structural gene of the target protein or polypeptide. As used herein, a DNA fragment having a nucleotide sequence lacking a portion of any one of the above-mentioned nucleotide sequences is intended to mean a DNA fragment which has functions relating to transcription, translation, and secretion of the gene, without having a portion of any one of the above-mentioned nucleotide sequences.

The recombinant microorganism of the present invention can be obtained by a conventional transformation technique in which a recombinant plasmid containing a DNA fragment which includes a gene encoding the target protein or polypeptide, and is ligated to a proper plasmid vector is transferred into a host microorganism cell. Alternatively, the recombinant microorganism may be obtained making use of a DNA fragment prepared by ligating the above DNA fragment to a proper region which is homologous with a certain portion of the host microorganism genome, and inserted directly into a host microorganism genome.

The target protein or polypeptide obtained by use of the recombinant microorganism of the present invention may be produced in such a manner that a corresponding cell strain is inoculated onto a culture medium containing assimilable carbon sources and nitrogen sources, and other essential components; the cell strain is cultured through a conventional microorganism culturing method; and subsequently, protein or polypeptide is collected and purified.

Through the aforementioned procedure, a host mutant microorganism strain in which any of the *Bacillus subtilis* genes shown in Table 1 or one or more genes selected from genes functionally equivalent thereto have been deleted or knocked out can be engineered. In addition, by use of such a mutant strain, a recombinant microorganism can be produced. Thus, a useful protein or polypeptide can be effectively produced through employment of the mutant strain or the recombinant microorganism.

The method for constructing a recombinant microorganism according to the present invention, and the method for producing cellulase and α-amylase by use of the recombinant microorganism will next be described in detail, centering on working examples for constructing recombinant strain belonging to *Bacillus subtilis* from which the ccpA gene (BG10376) of *Bacillus subtilis* has been deleted.

### Examples

### Example 1

A genome DNA sample, serving as a template, extracted from *Bacillus subtilis* 168 strain and two primer sets (ccpA-AF and ccpA-A/CmR; and ccpA-B/CmF and ccpA-BR) shown in Table 2 were used to prepare a 0.6 kb fragment (A) flanking the upstream side of the *ccpA* gene on the genome and a 0.6 kb fragment (B) flanking the downstream side of the *ccpA* gene. A chloramphenicol-resistant gene of plasmid pC194 (J. Bacteriol. 150 (2), 815 (1982))) was inserted into the XbaI-BamHI cleavages site of plasmid pUC18, to thereby prepare a recombinant plasmid pCBB 31. The recombinant plasmid pCBB and a primer set consisting of CmF and CmR shown in Table 2 were used to prepare a 1 kb fragment (C) containing the chloramphenicol-resistant gene. Subsequently, SOE-PCR was performed by use of the primers ccpA-AF and ccpA-BR shown in Table 2, and by use of the thus-prepared three fragments (A), (B), and (C) in combination as templates, a 2.2 kb DNA fragment in which the fragments (A), (B), and (C) were ligated in this sequence was prepared (see Fig. 1). By use of the thus-prepared DNA fragment, *Bacillus subtilis* 168 strain was transformed through the competent method. Colonies grown in an LB agar medium containing chloramphenicol were collected as transformants. The genome of the above-obtained transformant was extracted, and PCR performed thereon confirmed that the *ccpA* gene had been deleted and substituted by a chloramphenicol-resistant gene.

**Table 2-1**

| Primer | Nucleotide sequence | SEQ ID NO: |
|---|---|---|
| comA-AF | AAGGATGATAATCCGTCCCGTG | 7 |
| comA-A/CmR | GTTATCCGCTCACAATTCGGATGGTCATCAATCACTAG | 8 |
| comA-B/CmF | CGTCGTGACTGGGAAAACTGCGAAATCAGACGGTGTAC | 9 |
| comA-BR | CGTCGCCTATCGGCGGGCAC | 10 |
| yopO-AF | ATGTATATAGGAGGTTGGTGGTATG | 11 |
| yopO-A/CmR | GTTATCCGCTCACAATTCGCTCTGACATGTCAACCTCC | 12 |
| yopO-B/CmF | CGTCGTGACTGGGAAAACAGATGAGAAAGGAGGAGAAG | 13 |
| yopO-BR | ATAACTGTTACTATATAATGGCC | 14 |
| treR-AF | GCTGGGGATGACGAATCCGA | 15 |
| treR-A/CmR | GTTATCCGCTCACAATTCTCACCTTCATTATGGACCAC | 16 |
| treR-B/CmF | CGTCGTGACTGGGAAAACCACCGTCTCGACAAATTCCG | 17 |
| treR-BR | GTTGCCAAGCGCGATATAGG | 18 |
| yvbA-AF | TATACAGGGATTATCAGTATTGAGC | 19 |
| yvbA-A/CmR | GTTATCCGCTCACAATTCTTTTCTCCTTGTTGGATCTG | 20 |
| yvbA-B/CmF | CGTCGTGACTGGGAAAACGGGGATAACGATTTATGAAG | 21 |
| yvbA-BR | TTTTGTAATAATGATATGAAGCTAGTGTTG | 22 |
| cspB-AF | ATATCCAGCCCTGCCTCTTC | 23 |
| cspB-A/CmR | | 24 |
| cspB-B/CmF | | 25 |
| cspB-BR | TCCTGTTTGGGCTCCTGTTG | 26 |
| yvaN-AF | TGTTTATGTATGGCGGCCTGCGGGAC | 27 |
| yvaN-A/CmR | GTTATCCGCTCACAATTCAGCTTTCCATATATCTCACC | 28 |
| yvaN-B/CmF | CGTCGTGACTGGGAAAACACGGTCTGCTGATGACTGAC | 29 |
| yvaN-BR | GCGTTTACTTAAGATGTCGA | 30 |
| yttP-AF | TTTCTAGCGTTTCGGCAAATTGAGTTAAG | 31 |
| yttP-A/CmR | GTTATCCGCTCACAATTCCTTACTTTCATACGGCTCAC | 32 |
| yttP-B/CmF | CGTCGTGACTGGGAAAACGAGACGTGGCGCTCACCAAC | 33 |
| yttP-BR | CGGATTAAAAAAAGAATATCGCGGACAGC | 34 |
| yurK-AF | TGCCGCTGCCCGCCGGAGAG | 35 |

**Table 2-2**

| | | |
|---|---|---|
| yurK-A/CmR | GTTATCCGCTCACAATTCAAGGTGTAGAACTTCCGTTG | 36 |
| yurK-B/CmF | CGTCGTGACTGGGAAAACACCATCAACAGCCCCTACAC | 37 |
| yurK-BR | TCAAATAAAGGCGGCATTCAGTCC | 38 |
| yozA-AF | ATAATGGTATCCAAATCCACGC | 39 |
| yozA-A/CmR | GTTATCCGCTCACAATTCATTCAGTCATATGTATCACC | 40 |
| yozA-B/CmF | CGTCGTGACTGGGAAAACGATCCATCATACACAGCATG | 41 |
| yozA-BR | CACTTCTCAACGGAGGGGATTTCACATC | 42 |
| licR-AF | TAATGGAGGAGAGAAGGCCG | 43 |
| licR-A/CmR | GTTATCCGCTCACAATTCAGTCGCCCATGAAGCATGAG | 44 |
| licR-B/CmF | CGTCGTGACTGGGAAAACACCAAAAAATGCTGAGCTGACAGC | 45 |
| licR-BR | TTGCCAATGATGAGGAAAAAGGAACC | 46 |
| sigL-AF | CTGAACGTCTTGAATAAAAAAGCAGG | 47 |
| sigL-A/CmR | GTTATCCGCTCACAATTCGCTGAAGTTTCATATCCATC | 48 |
| sigL-B/CmF | CGTCGTGACTCGGAAAACATTCCGTCATCGGCAGCGAG | 49 |
| sigL-BR | AGCGGTTTACAAGTTGGAGG | 50 |
| mntR-AF | ATTTCAGAAGGCATACTTCAAG | 51 |
| rnntR-A/CmR | GTTATCCGCTCACAATTCCATACTTGGTGTTGTCATCG | 52 |
| mntR-B/CmF | CGTCGTGACTGGGAAAACCATAATCAGTAAAAAGGCGGTC | 53 |
| mntR-BR | TTCTGACCGCTCTGGCAACC | 54 |
| glcT-AF | ATAATGCCCGCTTCCCAACC | 55 |
| glcT-A/CmR | GTTATCCGCTCACAATTCCGATCCTCAGCTCCTTTGTC | 56 |
| glcT-B/CmF | CGTCGTGACTGGGAAAACTCATCTGATACCGATTAACC | 57 |
| glcT-BR | CAACTGAATCCGAAGGAATG | 58 |
| yvdE-AF | TCGGGGTCATGCCGAGCGGT | 59 |
| yvdE-A/CmR | GTTATCCGCTCACAATTCCAATGTTGCCATTTTCATCC | 60 |
| yvdE-B/CmF | CGTCGTGACTGGGAAAACTTGTACGAGAATCAACGCTG | 61 |
| yvdE-BR | CACGGCAATGCATTCTTCGG | 62 |
| ykvE-AF | AGATCTGTCGGCCAGGTTTAC | 63 |
| ykvE-A/CmR | GTTATCCGCTCACAATTCTGATTTTTCTGTCATGTCTC | 64 |
| ykvE-B/CmF | CGTCGTGACTGGGAAAACGGTAGAGATGTGCACCGAAA | 65 |
| ykvE-BR | GAGTCAGACGGCATCGATGA | 66 |
| slr-AF | TTCTGATTCATTTTCACTGCTGG | 67 |
| slr-A/CmR | GTTATCCGCTCACAATTCAACGGATAATTCTTCCAATC | 68 |
| slr-B/CmF | CGTCGTGACTGGGAAAACTGTCCATGAAGTCAAATCC | 69 |
| slr-BR | CGCTGAAATATTCTCTCGCA | 70 |
| rocR-AF | CGCCGCTTTCACCGCGGATTC | 71 |
| rocR-A/CmR | GTTATCCGCTCACAATTCCTTTGACCACTGTATGAACC | 72 |

**Table 2-3**

| | | |
|---|---|---|
| rocR-B/CmF | CGTCGTGACTGGGAAAACACTCGTCTAACGAATAATCC | 73 |
| rocR-BR | TGTCATCACGGAATTTGACG | 74 |
| ccpA-AF | CCAAATTATCCTTTGTGAGCGCGGAATCAG | 75 |
| ccpA-A/CmR | GTTATCCGCTCACAATTCCGTAGATCGTAATATTGCTC | 76 |
| ccpA-B/CmF | CGTCGTGACTGGGAAAACAGCTTAGAAAGTCAACCAAG | 77 |
| ccpA-BR | TTTGAGCATCAGCACAAGCC | 78 |
| yaaT-AF | TGTAGCAGAAGCAGTCGAATT | 79 |
| yaaT-A/Cm2R | CTAATGGGTGCTTTAGTTGACAATTACGCAGCTGTCATGT | 80 |
| yaaT-B/Cm2F | CTGCCCCGTTAGTTGAAGAACTGATAAACCGTGAAAAAGTG | 81 |
| yaaT-RV | CCTTTGAAAAAGGCTCCCGT | 82 |
| yyaA-AF | GTTTTCCAAGTCTGCCGATAAAAATATGC | 83 |
| yyaA-A/CmR | GTTATCCGCTCACAATTCATGCTTCATGTACCTACACC | 84 |
| yyaA-B/CmF | CGTCGTGACTGGGAAAACCAATTAACGATTCGCATACC | 85 |
| yyaA-BR | AAAAAGAAGAAGTCACAGTACAGAACGTGG | 86 |
| yycH-AF | ATTTTTCGCCATCTTGAATTTTC | 87 |
| yycH-A/Cm2R | CTAATGGGTGCTTTAGTTGGATGATCCTCTCGTTGAACTG | 88 |
| yycH-B/Cm2F | CTGCCCCGTTAGTTGAAGGGATGAGCCTTCAGAAAAGTT | 89 |
| yycH-BR | GCCGGACAGAGATCTGTATG | 90 |
| yacP-B/Cm4F | GAAGAAGGTTTTTATGTTGACGCTTTTTTGCCCAATACTGTATAA | 91 |
| yacP-B/Cm4R | CAAAAAAGCGTCAACATAAAAACCTTCTTCAACTAACGGGGCAGG | 92 |
| yacP-BR | AAGACGAGTACTTTTCTCTCTAAATCACTT | 93 |
| yacP-AF | AACTCGATCAAATGGTGACAGGACAGCATC | 94 |
| yacP-A/Cm4F | GGAGAATAAAGACCCTCTTCAACTAAAGCACCCATTAGTTCAACA | 95 |
| yacP-A/Cm4R | TGCTTTAGTTGAAGAGGGTCTTTATTCTCCCACAGGGTTTCGTTT | 96 |
| hprK-B/Cm4F | TTTTTATATTACAGCGAGTTGGCGTTAAATGAATGAAGCGATAGA | 97 |
| hprK-B/Cm4R | ATTTAACGCCAACTCGCTGTAATATAAAAACCTTCTTCAACTAAC | 98 |
| hprK-BR | TTGATTGATGATAAATTCAGGCAGGTGCAG | 99 |
| hprK-AF | CAAAGCTTGAGAAATGTTCCCATGCTCTTG | 100 |
| hprK-A/Cm4F | CAGGAGGAACATATCTCTTCAACTAAAGCACCCATTAGTTCAACA | 101 |
| hgrK-A/Cm4R | TGCTTTAGTTGAAGAGATATGTTCCTCCTGTTCCGGGCTGCCCCG | 102 |
| rsiX-AF | ATTCCAGTTACTCGTAATATAGTTG | 103 |
| rsiX-A/CmR | GTTATCCGCTCACAATTCACTTCATCATCCATTAGCTC | 104 |
| rsiX-B/CmF | CGTCGTGACTGGGAAAACCTGCTCCAAATCCGATTTCC | 105 |
| rsiX-BR | GTCCTGCATTTTTCGAAGTCTGG | 106 |
| yhdK-AF | TACACATCCTTCAAACAAGTCTGAACAAAC | 107 |

**Table 2-4**

| | | |
|---|---|---|
| yhdK-A/Cm4R | TGCTTTAGTTGAAGATTACCAGTTCCATAATTCCACCTCGCCGAC | 108 |
| yhdK-B/Cm4F | TTTTTATATTACAGCGTGTGTATACCATTGTATCTGTAGATACGA | 109 |
| yhdK-BR | GCTATGATCATTGTAACGAAAGGAAAGGGG | 110 |
| yhdK-A/Cm4F | TTATGGAACTGGTAATCTTCAACTAAAGCACCCATTAGTTCAACA | 111 |
| yhdK-B/Cm4R | CAATGGTATACACACGCTGTAATATAAAAACCTTCTTCAACTAAC | 112 |
| ylbO-AF | AATCTGAACAAGAAAAAGGAGCTGCTCCTC | 113 |
| ylb0-A/Cm4R | TGCTTTAGTTGAAGAATTCAATCTCCCTCCATGTCAGCTTATTTA | 114 |
| ylb0-B/Cm4F | TTTTTATATTACAGCAGAAACGCCTGAAATGAACCGGCCCTATAG | 115 |
| ylbO-BR | TGTTTGACAAAGGTAGAACGTCTGCTTATC | 116 |
| ylbO-A/Cm4F | GGAGGGAGATTGAATTCTTCAACTAAAGCACCCATTAGTTCAACA | 117 |
| ylbO-B/Cm4R | ATTTCAGGCGTTTCTGCTGTAATATAAAAACCTTCTTCAACTAAC | 118 |
| CmF | GAATTGTGAGCGGATAAC | 119 |
| CmR | GTTTTCCCAGTCACGACG | 120 |
| Cm2F | CAACTAAAGCACCCATTAG | 121 |
| Cm2R | CTTCAACTAACGGGGCAG | 122 |

### Example 2

In a manner similar to that described in Example 1, sporulation gene-deleted strains into which a chloramphenicol-resistant gene had been introduced by way of substitution in place of the below-described deleted genes were separated through use of a DNA fragment for effecting deletion prepared from an adequate primer set selected from among various primer sets shown in Table 2; i.e., gene-AF, gene-A/CmR, gene-B/CmF, gene-BR, CmF, and CmR. The gene deleted from the genome was *comA, yopO, treR, yvbA, yvaN, yttP, yurK, yozA, licR, sigL, mntR, glcT, ykvE, slr, rocR, yyaA,* or *rsiX*.

### Example 3

In a manner similar to that described in Example 2, a DNA fragment for deletion was prepared by use of an adequate primer set selected from among the gene-AF, gene-A/Cm2R, gene-B/Cm2F, gene-BR, Cm2F, and Cm2R, which are shown in Table 2. By use of the thus-prepared DNA fragment, sporulation gene-deleted strains into which a chloramphenicol-resistant gene had been introduced by way of substitution in place of the below-described deleted genes were separated. The gene deleted from the genome was *cspB, yvdE, yaaT, yycH,* or *ylbO.*

### Example 4

In a manner similar to that described in Example 2, a DNA fragment for effecting deletion was prepared from an adequate primer set selected from among the gene-AF, gene-A/Cm4R, gene-B/Cm4F, gene-BR, Cm4F, and Cm4R, which are shown in Table 2. By use of the thus-prepared DNA fragment, sporulation gene-deleted strains into which a chloramphenicol-resistant gene had been introduced by way of substitution in place of deleted genes; *yacP, hprK,* and yhdK, were separated.

### Example 5

To each of the gene-deleted strains obtained in Examples 1 to 4 and to *Bacillus subtilis* 168 strain serving as a control, a recombinant plasmid pHY-S237 was introduced through the protoplast transformation method. The recombinant plasmid pHY-S237 was prepared by inserting a DNA fragment (3.1 kb) encoding an alkaline cellulase derived from *Bacillus* sp. KSM-S237 strain (Japanese Patent Application Laid-Open *(kokai)* No. 2000-210081) into the restriction enzyme *Bam*HI cleavage site of a shuttle vector pHY300 PLK. Each of the thus-obtained cell strains was shake-cultured in an LB medium (5 mL) overnight at 30°C. The culture broth (0.03 mL) was inoculated to a 2 × L-maltose medium (2% tryptone, 1% yeast extract, 1% NaCl, 7.5% maltose, 7.5 ppm manganese sulfate 4-5 hydrate, and 15 ppm tetracycline), followed by shake culturing at 30°C for three days. After completion of culturing, cells were removed through centrifugation, and alkaline cellulase activity of the supernatant obtained from the culture was determined, thereby calculating the amount of the alkaline cellulase secreted from the cells during culturing; i.e., the amount of the extracellularly produced alkaline cellulase. As is clear from Table 3, more effective production, or secretion, of alkaline cellulase has been confirmed in the case where a gene-deleted strain was employed as a host, as compared with the control 168 strain (wild type strain).

**Table 3**

| Name of deleted gene | Gene ID | Gene size (bp) | Size of deleted fragment (bp) | Amount of produced (secreted) alkaline cellulase (relative value) |
|---|---|---|---|---|
| *ComA* | BG10381 | 645 | 588 | 160 |
| *yopO* | BG13648 | 213 | 169 | 154 |
| *treR* | BG11011 | 717 | 656 | 139 |
| *yvbA* | BG14078 | 273 | 210 | 137 |
| *cspB* | 8G10824 | 204 | 171 | 132 |
| *yvaN* | BG14069 | 408 | 379 | 124 |
| *yttP* | BG13927 | 624 | 590 | 121 |
| *yurK* | BG13997 | 729 | 677 | 118 |
| *yozA* | BG13748 | 324 | 289 | 117 |
| *licR* | BG11346 | 1926 | 1889 | 116 |
| *sigL* | BG10748 | 1311 | 1256 | 114 |
| *mntR* | BG11702 | 429 | 399 | 114 |
| *glcT* | BG12593 | 858 | 811 | 110 |
| *yvdE* | BG12414 | 951 | 916 | 109 |
| *ykvE* | BG13310 | 438 | 356 | 108 |
| *slr* | BG11858 | 459 | 394 | 105 |
| *rocR* | BG10723 | 1386 | 1359 | 128 |
| *ccpA* | BG10376 | 1005 | 957 | 205 |
| *yaaT* | BG10096 | 828 | 828 | 127 |
| *yyaA* | BG10057 | 852 | 816 | 113 |
| *yycH* | BG11462 | 1368 | 1368 | 146 |
| *yacP* | BG10158 | 513 | 513 | 156 |
| *hprK* | BG14125 | 933 | 933 | 196 |
| *rsiX* | BG10537 | 1107 | 1068 | 125 |
| *yhdK* | BG13017 | 291 | 228 | 114 |
| *ylb0* | BG13367 | 582 | 582 | 136 |
| None (Wild type) | - | - | - | 100 |

### Example 6

To each of the gene-deleted strains obtained in Examples 1 to 4 and to *Bacillus subtilis* 168 strain serving as a control, recombinant plasmid pHSP-K38 was introduced through the protoplast transformation method. The recombinant plasmid pHSP-K38 was prepared by inserting, into the restriction enzyme BagII-XbaI cleavage site of a shuttle vector pHY300 PLK, a 2.1 kb fragment (SEQ ID No: 5) prepared by ligating an upstream 0.6 kb fragment (SEQ ID NO: 3) including portions of a promoter region and a signal sequence region of an alkaline cellulase gene with an upstream side of a DNA fragment (1.5 kb) encoding a mature enzyme region (Asp1-Gln480) of an alkaline amylase gene derived from *Bacillus* sp. KSM-K38 strain (Japanese Patent Application Laid-Open (*kokai*) No. 2000-1884882, Eur. J. Biochem., 268, 2974 (2001)). Each of the thus-obtained cell strains was shake-cultured in an LB medium (5 mL) overnight at 30°C. The culture broth (0.03 mL) was inoculated to a 2 × L-maltose medium (2% tryptone, 1% yeast extract, 1% NaCl, 7.5% maltose, 7.5 ppm manganese sulfate 4-5 hydrate, and 15 ppm tetracycline), followed by shake culturing at 30°C for three to six days. After completion of culturing, cells were removed through centrifugation, and alkaline amylase activity of the supernatant obtained from the culture was determined, thereby calculating the amount of the alkaline amylase secreted from the cells during culturing; i.e., the amount of the extracellularly produced alkaline amylase. As is clear from Table 3, more effective production, or secretion, of alkaline amylase has been confirmed in the case where a gene-deleted strain was employed as a host, as compared with the control 168 strain (wild type strain).

**Table 4**

| Name of deleted gene | Gene ID | Gene size (bp) | Size of deleted fragment (bp) | Amount of produced (secreted) alkaline amylase (relative value) |
|---|---|---|---|---|
| Cultured for 3 days | | | | |
| *slr* | BG11858 | 459 | 394 | 178 |
| *treR* | BG11011 | 717 | 656 | 124 |
| *yop0* | BG13648 | 213 | 169 | 364 |
| *yvaN* | BG14069 | 408 | 379 | 148 |
| *yvbA* | BG14078 | 273 | 210 | 171 |
| None (Wild type) | - | - | - | 100 |

| Culture for 5 days (Wild type) | | | | |
|---|---|---|---|---|
| *cspB* | BG10824 | 204 | 171 | 195 |
| *rocR* | BG10723 | 1386 | 1359 | 215 |
| *sigL* | BG10748 | 1311 | 1256 | 204 |
| *glcT* | BG12593 | 858 | 811 | 132 |
| *yvdE* | BG12414 | 951 | 916 | 127 |
| *yacP* | BG10158 | 513 | 513 | 110 |
| None (Wild type) | - | - | - | 100 |

| Cultured for 6days | | | | |
|---|---|---|---|---|
| *yycH* | BG11462 | 1368 | 1368 | 120 |
| *licR* | BG11346 | 1926 | 1889 | 122 |
| None (Wild type) | - | - | - | 100 |

## Claims

1. A recombinant microorganism prepared by transferring, to a mutant strain of a microorganism from which any of *Bacillus subtilis* genes cspB, sigL, glcT, rocR and ccpA have been deleted or knocked out, a gene encoding a heterologous protein or polypeptide,
wherein the microorganism is *Bacillus subtilis* ,wherein three regions constituted by a transcription initiation regulatory region, a translation initiation regulatory region, and a secretion signal region are ligated to an upstream region of the gene encoding a heterologous protein or polypeptide, wherein the transcription initiation regulatory region and the translation initiation regulatory region are each derived from a 0.6 to 1 kb region upstream of the cellulase gene of a bacterium belonging to the genus Bacillus, and wherein the three regions constituted by the transcription initiation regulatory region, the translation initiation regulatory region, and the secretion signal region are
(a) a nucleotide sequence of base numbers 1 to 659 of a cellulase gene of SEQ ID NO: 1;
(b) a nucleotide sequence of base numbers 1 to 696 of a cellulase gene of SEQ ID NO: 3; or
(c) a DNA fragment having a nucleotide sequence having 80% homology with either of the nucleotide sequences of (a) or (b).

2. A method for producing a protein or polypeptide by use of a recombinant microorganism as defined in claim 1.

## Patentansprüche

1. Rekombinanter Mikroorganismus, hergestellt durch Transfer eines Gens, das ein heterologes Protein oder Polypeptid codiert, in einen Stamm einer Mutante eines Mikroorganismus, aus dem ein beliebiges der *Bacillus subtilis-Gene* cspB, sigL, glcT, rocR und ccpA deletiert oder inaktiviert wurde,
wobei der Mikroorganismus *Bacillus subtilis* ist, wobei drei Regionen, die aus einer die Initiation der Transkription regulierenden Region, einer die Initiation der Translation regulierenden Region und einer Sekretionssignalregion bestehen, an eine upstream-Region des Gens, welches ein heterologes Protein oder Polypeptid codiert, ligiert sind, wobei die die Initiation der Transkription regulierende Region und die die Initiation der Translation regulierende Region beide aus einer Region stammen, die 0,6 kb bis 1 kb upstream des Cellulasegens eines Bakteriums liegen, das zur Gattung Bacillus gehört, und wobei die drei Regionen, die aus der die Initiation der Transkription regulierenden Region, der die Initiation der Translation regulierenden Region und der Sekretionssignalregion bestehen,
(a) eine Nucleotidsequenz gemäß der Basen 1 bis 659 eines Cellulasegens der SEQ ID NO:1;
(b) eine Nucleotidsequenz gemäß der Basen 1 bis 696 eines Cellulasegens der SEQ ID NO:3; oder
(c) ein DNA-Fragment mit einer Nucleotidsequenz, die 80% Homologie mit einer der Nucleotidsequenzen von (a) oder (b) hat,
sind.

2. Verfahren zur Herstellung eines Proteins oder Polypeptids unter Verwendung eines wie in Anspruch 1 definierten rekombinanten Mikroorganismus.

## Revendications

1. Micro-organisme recombinant préparé par transfert, dans une souche mutante d'un micro-organisme duquel l'un quelconque des gènes cspB, sigL, glcT, rocR et ccpA de *Bacillus subtilis* a été délété ou invalidé, d'un gène codant pour une protéine ou un polypeptide hétérologue,
dans lequel le micro-organisme est *Bacillus subtilis,* dans lequel trois régions constituées par une région régulatrice de l'initiation de la transcription, une région régulatrice de l'initiation de la traduction, et une région signal de sécrétion sont ligaturées à une région en amont du gène codant pour une protéine ou un polypeptide hétérologue, dans lequel la région régulatrice de l'initiation de la transcription et la région régulatrice de l'initiation de la traduction sont chacune dérivées d'une région de 0,6 à 1 kb en amont du gène de la cellulase d'une bactérie appartenant au genre Bacillus, et dans lequel les trois régions constituées par la région régulatrice de l'initiation de la transcription, la région régulatrice de l'initiation de la traduction, et la région signal de sécrétion sont
(a) une séquence de nucléotides représentée par les numéros de bases 1 à 659 d'un gène de la cellulase de SEQ ID No : 1 ;
(b) une séquence de nucléotides représentée par les numéros de bases 1 à 696 d'un gène de la cellulase de SEQ ID No : 3 ; ou
(c) un fragment d'ADN ayant une séquence de nucléotides présentant une homologie de 80 % avec l'une ou l'autre des séquences de nucléotides de (a) ou (b).

2. Méthode de production d'une protéine ou d'un polypeptide utilisant un micro-organisme recombinant tel que défini dans la revendication 1.
